(19) Europäisches Patentamt
European Patent Office
Office européen des brevets

(11) **EP 3 426 315 B1**

(12) **EUROPEAN PATENT SPECIFICATION**

(45) Date of publication and mention
of the grant of the patent:
**12.02.2025 Bulletin 2025/07**

(21) Application number: **17715299.8**

(22) Date of filing: **13.03.2017**

(51) International Patent Classification (IPC):
*A61L 31/14* (2006.01)        *A61L 31/16* (2006.01)

(52) Cooperative Patent Classification (CPC):
**A61L 31/148; A61L 31/14; A61L 31/16**

(86) International application number:
**PCT/US2017/022090**

(87) International publication number:
**WO 2017/156530 (14.09.2017 Gazette 2017/37)**

(54) **PARTIALLY DEGRADABLE STENTS FOR CONTROLLED REDUCTION OF INTRAOCULAR PRESSURE**

TEILWEISE ABBAUBARE STENTS ZUR KONTROLLIERTEN VERRINGERUNG DES AUGENINNENDRUCKS

STENTS PARTIELLEMENT DÉGRADABLES POUR RÉDUCTION CONTRÔLÉE DE LA PRESSION INTRAOCULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.03.2016 US 201662306848 P**

(43) Date of publication of application:
**16.01.2019 Bulletin 2019/03**

(73) Proprietor: **The Johns Hopkins University Baltimore, MD 21218 (US)**

(72) Inventors:
• **PARIKH, Kunal, S.**
  **Reynoldsburg, OH 43068 (US)**
• **PITHA, Ian**
  **Baltimore, MD 21212 (US)**
• **HANES, Justin**
  **Baltimore, MD 21212 (US)**

(74) Representative: **Potter Clarkson**
**Chapel Quarter**
**Mount Street**
**Nottingham NG1 6HQ (GB)**

(56) References cited:
**WO-A1-2013/184538     WO-A2-99/33410**

# EP 3 426 315 B1

## Description

This invention was made with government support under grant numbers BGE-1232825, K08EY024952, and UL1 TR 001079, awarded by the National Science Foundation, National Institutes of Health, and National Center for Advancing Translational Sciences, respectively. The government has certain rights in the invention.

## FIELD OF THE INVENTION

The present invention relates to the field of biodegradable stents, and more particularly, to tube-shunt implants.

## BACKGROUND OF THE INVENTION

Glaucoma is a progressive optic neuropathy characterized by changes to the optic nerve and visual field loss. It is the second-leading cause of irreversible blindness and affects more than 60 million people worldwide (Quigley HA and Broman AT, Br J Ophthalmol, 90(3):262-267 (2006); Tham Y-C, et al., Ophthalmology, 121(11):2081-90 (2014)). The elevation of intraocular pressure (IOP) is the major modifiable risk factor of glaucoma and, if left untreated, results in disease progression in 70 % of all glaucoma cases (Saccà SC, et al., Chapter 4 in Handbook of Nutrition, Diet and the Eye. San Diego: Academic, p. 29-40 (2014)). According to the Early Manifest Glaucoma Treatment trial, for every 1-mm Hg reduction in IOP, the risk of glaucoma progression is decreased by 10 % (Leske MC, et al., Ophthalmology, 106(11):2144-53 (1999)).

Traditional therapies to reduce IOP include topical eye drop medications, laser procedures, and incisional surgeries. In many cases such as congenital glaucoma, the less invasive approaches to IOP reduction are not sufficient or not practical, and incisional surgical approach is frequently performed. Surgeries such as the trabeculectomy and glaucoma drainage implant (GDI) insertion create a passage between the anterior chamber and the subconjunctival space, allowing for aqueous humor to vent and fluid outflow into the subconjunctival space (Gedde SJ, et al., Am J Ophthalmol. 148(5):670-684 (2009)). When such a passage is created, IOP is regulated by the combined outflow resistance through the outflow track (or shunt) and out of the subconjunctival space.

However, controlling the degree of IOP reduction is a significant challenge, and excessive pressure reduction results in hypotony, i.e., lower than normal IOP. For example, low IOPs (hypotony), as well as flat anterior chambers, can occur after trabeculectomy (up to 18%) or following the insertion of GDIs such as the Ahmed and Baerveldt implants (Gedde SJ, et al., Am J Ophthalmol., 143(1):23-31(2007); Solus JF, et al., Ophthalmology, 119(4):703-711(2012); Olayanju JA, et al., JAMA Ophthalmol., 133(5):574-580 (2015)). Early post-operative hypotony occurs due to the relatively little resistance to outflow provided by the subconjunctival space in the immediate post-operative period. The Collaborative Initial Glaucoma Treatment Study (CIGTS) randomized glaucoma patients to either eye drops or surgery (Feiner L, Piltz-Seymour JR, Curr Opin Ophthalmol., 14(2):106-111 (2003)). While surgery lowered IOP more effectively than eye drop treatment, it was associated with reduced visual acuity, increased cataract formation, and perioperative complications such as hyphema (bleeding in the eye), ptosis (droopy eyelid), and hypotony - which each occurred in approximately 10% of cases (Jampel HD, et al., Am J Ophthalmol., 140(1):16-22 (2005).

Although post-operative healing is able to provide outflow resistance in the reduction of IOP, as is mostly relied upon by traditional approaches, vigorous healing with a robust scarring process (e.g., fibrosis) in the subconjunctival space can cause surgical failure, where the outflow resistance into the subconjunctival space becomes too high. The five-year failure rates for trabeculectomy, Ahmed implant, and Baerveldt implant are 25.7%, 39.5%, and 44.7%, respectively (Solus JF, et al., Ophthalmology, 119(4):703-711(2012); Budenz DL, et al., Ophthalmology, 122(2):308-316

(2015)). These devices are still vulnerable to inconsistency, and they still render early post-operative, excess exposure of the conjunctiva to aqueous humor, where the elevated levels of TGF-β from aqueous humor can promote vigorous conjunctival scarring (Cheng J, et al., J Glaucoma, 24(4):e34-5 (2015); Freedman J, Ophthalmology, 116(1):166 (2009)), leading to higher rates of surgical failure. Furthermore, variations in surgical techniques performed by surgeons, as well as reliance on surgical "fixes", lead to variability in clinical outcomes. (Examples of surgical "fixes" include absorbable sutures in Baerveldt GDI insertion and laser suture lysis in trabeculectomy, in order to overcome the flaws in surgical and device design.)

WO 2013/184538 was published on 12 December 2013 and discloses an implantable device which includes a tubular fiber wall defining a lumen adapted to allow the passage of fluid.

WO 99/33410 was published on 8 July 1999 and discloses biodegradable polymeric stents having a programmed pattern of *in vivo* degradation.

Therefore, it is an object of the present invention to provide a device to predictably and controllably reduce IOP and provide long-term post-operative control of IOP.

It is another object of the present invention to provide a device to control the drainage of aqueous humor, and preferably deliver agents locally during the post-operative healing process.

It is yet another object of the present invention to provide a method of preventing, inhibiting and treating glaucoma and other ophthalmic disorders

The references to methods of treatment in the summary and detailed description of the invention in this description are to be interpreted as references to the devices of the present invention for use in a method for treatment of the human (or animal) body by therapy (or for diagnosis).

## SUMMARY OF THE INVENTION

The invention is limited to the scope of the appended claims.

The present disclosure discloses a tubular device having a biodegradable inner core and a non-degradable or slowly degradable outer layer, and optionally delivering therapeutic, prophylactic, and/or diagnostic agents, has been developed. The degradable core can result in a device in which the lumen diameter increases over time as a function of degradation. Optionally, the device contains an exterior coating, which in some embodiments is fluid-impermeable, releases a therapeutic and/or prophylactic agent, is degradable, and/or improves the glideability (e.g., by inclusion of glyconate). The device may include micro-or nano-fibers which are woven around a rotating cylindrical template. Removal of the template results in a tubular device with a lumen. Devices made with nano-structured fibers reduce protein adsorption and *in vivo* inflammatory response. The present invention is an implantable device as defined in the claims.

In some embodiments, the implantable device is formed by electrospinning polymer solutions to allow deposition of a wide range of polymer in the form of polymeric nanofibers and agents in user-determined dimensions and surface properties. Since the degradable polymeric nanofibers are positioned in the inner portion of the formed device and the non-degradable or slower degradable polymeric nanofibers are positioned in the outer portion of the device, the degradation of the inner core leads to an increase in the internal diameter of the device after implantation *in vivo.*

This type of implantable device is referred to as a "pressure control shunt", having a biodegradable core to provide resistance to flow immediately after surgery (e.g., to avoid post-surgery hypotony) and provide long-term pressure reduction following core degradation (e.g., to prevent and mitigate elevated intraocular pressure). The rate of the degradation is controlled by selecting the chemical structure, molecular weight, and linear/branching structure of the degradable polymer, as well as its concentration/density, alignment and degree of interpenetration with the non-degradable fibers in the formed device, and fiber size and porosity. The biodegradable core of the device typically degrades entirely over a period between 14 days and 100 days, preferably between two weeks to six weeks, allowing for gradual enlargement of the lumen of the device. The luminal core typically has an initial averaged diameter between about 50 $\mu$m and about 100 $\mu$m, preferably between about 50 $\mu$m to about 80 $\mu$m, and more preferably of about 50 $\mu$m, whereas its end diameter after the degradation of the inner portion is between about 50 $\mu$m and about 100 $\mu$m, preferably between about 60 $\mu$m and about 80 $\mu$m.

The implantable devices may be prepared with non-swellable materials in the shape of nano- and/or micro-fibers. In one embodiment, the device is a tube. In another embodiment, the device is a stent. Yet in another embodiment, the device is a vessel.

The mechanical properties, e.g., the strength, flexibility and hardness of the electrospun fibers forming the implantable device, is tuned using a heat treatment above the glass transition temperature of polymer.

In preferred embodiments, the device is used for reducing the intraocular pressure (IOP) in a patient by facilitating drainage of aqueous humor, without the use of surgical "fixers" such as sutures. It can be implanted to connect the anterior chamber to the subconjunctival space or to the suprachoroidal space of an eye to permit outflow or venting of aqueous humor, providing immediate pressure decrease following surgical implantation. As the lumen diameter is relatively small in the beginning and enlarges over time with the passive degradation of the inner portion of device, a controlled pressure reduction is achieved, avoiding the occurrence of hypotony. A non-degradable outer layer of the device is "permanent", and provides a relatively long-term decrease of IOP even after the inner portion is completely degraded. A degradable outer layer of the device at a slower degradation rate than the inner core provides a fully absorbable device. In some embodiments where fully absorbable device is desired in applications, the degradation of the outer wall of the device may be timed to coincide with the healing of the surrounding tissue. The diameter and the length of the lumen are factors affecting the differential pressure of fluid passing through the device. In some embodiments, the pressure drop of the fluid through the tubular device is about 17 mm Hg before the enlargement of the luminal core, and about 5 mm Hg after the enlargement of the luminal core. The pressure difference along the device as predicted based on a simulation using the Hagen-Poiseuille (HP) equation is consistent with experimental data as the lumen diameter increases. Generally, the device when implanted for prevention and/or treatment of glaucoma prevents hypotony after implantation and facilitates venting of intraocular pressure in a controlled manner.

In some embodiments, the device is used in treating the symptoms of glaucoma or preventing the worsening of glaucoma. It can contain and deliver with a controlled release rate effective amounts of one or more active, such as anti-inflammatory agents, including immunosuppressant agents, anti-allergenic agents, anti-infectious agents, anti-fibrosis agents, anti-glaucoma agents and anesthetic agents.

3

Anti-fibrotic pressure control shunts, as well as fully absorbable drug-eluting shunts, with appropriate selection of polymer composition and therapeutic agents, reduce complications, improve long-term efficacy, and reverse abnormal function to restore normal outflow.

In other embodiments, the device can be used as a conduit or as a porous polymeric scaffold for the regeneration of peripheral nerve or vasculature.

## BRIEF DESCRIPTION OF THE DRAWINGS

Figure 1 is a schematic of the electrospinning configuration to make the tubular device.

Figure 2 is a schematic of partially degradable stent with an exemplary dimension, where the lumen diameter is A, the outer diameter of the "ring" formed by a first plurality of biodegradable polymer (or the inner diameter of the "ring" formed by a second plurality of polymer - herein non-degradable) is B, and the outer diameter (or overall diameter) of the stent is C.

Figure 3 is a line graph showing the predicted pressure difference ($\Delta P$, mm Hg) of an incompressible and Newtonian fluid, resulting from a laminar flow through a cylindrical pipe, over the radius (r, $\mu$m) of the pipe, according to the HP equation, for pipes of different lengths (mm) such as 25 mm, 20 mm, 15 mm, 10 mm, and 5 mm.

Figure 4 is a schematic of an exemplary cylindrical shunt model based on the Hagen-Poiseuille Equation (HPE) denoting the length, $L$, and a lumen cross-sectional diameter, $d$.

Figure 5A is a graph showing the pressure difference (mmHg) of an incompressible and Newtonian fluid at the entrance and at the exit of a cylindrical shunt based on a HPE model over a range of shunt lengths (mm) for each of shunt lumen diameters of 50, 75, 100, 125, and 150 $\mu$m, when the flow rate is 150 $\mu$L/h.

Figure 5B is a graph showing the pressure difference (mmHg) at the entrance and at the exit of a cylindrical shunt based on a HPE model over a range of flow rates ($\mu$L/hr) through the shunt for each of shunt lengths of 10, 8, 6, 4, and 2 mm.

Figures 6A, 6B, and 6C are graphs showing the differential pressure (mmHg) of a flow of phosphate buffered saline (PBS) at different flow rates ($\mu$L/hr) through a cylindrical shunt with a lumen diameter of 50 $\mu$m where the shunt is 5 mm (figure 6A), 6 mm (figure 6B), and 7 mm (figure 6C) in length, respectively. Experimental data is denoted by squares, and theoretical differential pressure according to the Hagen-Poiseuille Equation is denoted by solid black lines.

Figure 7A is a line graph showing intraocular pressure (mmHg) of rabbits over time (days) following implantation of closed (i.e., template wire remaining in the center of) polyethylene terephthalate (PET) shunts, compared to control contralateral eye (i.e., not implanted with shunt).

Figure 7B is a line graph showing intraocular pressure (mmHg) of rabbits over time (days) following implantation of open (i.e., center template wire removed from) polyethylene terephthalate (PET) shunts, compared to control contralateral eye (i.e., not implanted with shunt).

Figure 8A is a line graph showing the cumulative release of ethacrynic acid ($\mu$g) from a shunt over time (days).

Figure 8B is a line graph showing the intraocular pressure (mmHg) over time (days) following implantation of a fully absorbable shunt made from poly-L-lactic acid, where the shunt contains ethacrynic acid (PLLA/ETA) or doesn't contain ethacrynic acid (PLLA).

## DETAILED DESCRIPTION OF THE INVENTION

### I. Definitions

The terms "shunt", "stent", "stent device", and "tube", used herein, refer to a device allowing movement of fluid through the lumen of the device for controlled reduction of fluid pressure on one end of the device. In some embodiments, the device controls outflow of aqueous humor to realize a normal intraocular pressure. A trabecular shunting and stenting device is adapted for implantation within a trabecular meshwork of an eye such that aqueous humor flows controllably from an anterior chamber of the eye to Schlemm's canal, bypassing the trabecular meshwork. In some embodiments, the device permits movement of blood, acting as a duct to keep the passageway open.

The terms "venting resistance", "differential pressure", "pressure difference", used herein, refer to the difference in pressure between two points, and in some embodiments, the fluid pressure difference between the entrance and the exit of stent devices, e.g., devices for implantation to vent aqueous humor.

The term "degrade", as used herein, refers to a reduction in one or more properties of the polymer over time, molecular weight, total mass, mechanical strength, elasticity, and/or the density or porosity of the fibers formed from polymers. Degradable or biodegradable polymer is capable of being fully absorbed by living mammalian tissue. The prevailing mechanism of degradation of biodegradable polymer is chemical hydrolysis of the hydrolytically unstable backbone. In a bulk eroding polymer, the polymer network is fully hydrated and chemically degraded throughout the entire polymer volume. As the polymer degrades, the molecular weight decreases. The reduction in molecular weight is followed by a decrease in mechanical properties (e.g., strength) and scaffold properties. The decrease of mechanical properties is followed by loss of mechanical integrity and then erosion or mass loss (Pistner et al., Biomaterials, 14: 291-298 (1993)).

The term "biodegradable" as used herein, generally refers to a material that will degrade or erode under physiologic conditions to smaller units or chemical species that are capable of being metabolized, eliminated, absorbed, or excreted by the subject. The degradation time is a function of composition and morphology. Degradation times can be from hours to years. "Non-biodegradable" refers to a material that will not degrade within a time span under reasonable physiological (including pathological) environments *in vivo,* e.g., non-degradable materials does not fully absorb within the living mammalian tissue within two years.

The term "mechanical strength", as used herein, may be characterized by any one of the following: ultimate tensile strength (maximum stress borne until failure (N)), peak load, load at yield, tenacity, initial stiffness (N/mm), or the modulus of elasticity (Young's modulus). The modulus of elasticity measures an object or substance's resistance to being deformed elastically (i.e., non-permanently) when a force is applied to it. The elastic modulus of an object is defined as the slope of its stress-strain curve in the elastic deformation region. It can be measured using the following Formula: $E = Stress / Strain$, where *Stress* is the force causing the deformation divided by the area to which the force is applied and *Strain* is the ratio of the change in some length parameter caused by the deformation to the original value of the length parameter. The modulus of elasticity is presented in Pascals (Pa), megapascals (1 MPa = $10^6$ Pa), or gigapascals (1 GPa = $10^9$ Pa).

The term "swelling", herein in relation to "swelling of polymer", refers a polymer when placed in a solvent absorbs a portion of the solvent, rather than dissolving completely, and increases in the volume of material by, for example, 50% to 250% or greater. The term "non-swelling" refers to a polymer generally swells less than 25%, 10%, 8%, or 5% in a solvent. Herein a relevant solvent or medium includes physiological medium or aqueous medium.

The term "therapeutic agent" refers to an agent that can be administered to prevent or treat one or more symptoms of a disease or disorder. These may be a nucleic acid, a nucleic acid analog, a small molecule, a peptidomimetic, a protein, peptide, carbohydrate or sugar, lipid, or surfactant, or a combination thereof.

The term "diagnostic agent", as used herein, generally refers to an agent that can be administered to reveal, pinpoint, and define the localization of a pathological tissue, agent, or to image a desired process or tissue or agent.

The term "prophylactic agent", as used herein, generally refers to an agent that can be administered to prevent one or more symptoms of a disea, disorder or certain conditions like pregnancy.

The phrase "pharmaceutically acceptable" refers to compositions, polymers and other materials and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. The phrase "pharmaceutically acceptable carrier" refers to pharmaceutically acceptable materials, compositions or vehicles, such as a liquid or solid filler, diluent, solvent or encapsulating material involved in carrying or transporting any subject composition, from one organ, or portion of the body, to another organ, or portion of the body. Each carrier must be "acceptable" in the sense of being compatible with the other ingredients of a subject composition and not injurious to the patient.

The phrase "therapeutically effective amount" refers to an amount of the therapeutic agent that produces some desired effect at a reasonable benefit/risk ratio applicable to any medical treatment. The effective amount may vary depending on such factors as the disease or condition being treated, the particular targeted constructs being administered, the size of the subject, or the severity of the disease or condition. One of ordinary skill in the art may empirically determine the effective amount of a particular compound without necessitating undue experimentation.

The term "treating" refers to preventing or alleviating one or more symptoms of a disease, disorder or condition. Treating the disease or condition includes ameliorating at least one symptom of the particular disease or condition, even if the underlying pathophysiology is not affected, such as treating the pain of a subject by administration of an analgesic agent even though such agent does not treat the cause of the pain.

The term "biocompatible" as used herein, generally refers to materials that are, along with any metabolites or degradation products thereof, generally non-toxic to the recipient, and do not cause any significant adverse effects to the recipient. Generally speaking, biocompatible materials are materials which do not elicit a significant inflammatory or immune response when administered to a patient.

Electrospinning is a technique that employs electric forces to elongate and reduce the diameter of a viscoelastic polymer jet, allowing for the formation of solid fibers ranging from nanometers to microns in diameter. Electrospinning from molten precursors is also practiced; this method ensures that no solvent can be carried over into the final product. A polymeric material to be applied in electrospinning includes, but is not limited to, a polymer solution, a monomeric precursor thereof, sol-gel precursor, particulate suspension, and melt, It is described by Li D, et al., Advanced materials 16(14):1151-1170 (2004) and Bhardwaj N, et al., Biotechnol Adv., 28(3):325-347 (2010).

The term "grounded" generally refers to the status of connection to a ground. In electrical engineering, ground or earth is the reference point in an electrical circuit from which voltages are measured, a common return path for electric current, or a direct physical connection to the Earth. Therefore "grounded" as used herein in relation to electrospinning refers a collector acting as an electrode that is connected to ground or earth, as compared to a positive electrode (e.g., a charged needle tip or nozzle).

The term "collector" as used herein refers to a device where electrically charged solution, jet, melt, or gel is deposited onto in an electric field. Generally the collector is grounded, so as to provide a grounded electrode (that is apart from a positive electrode (e.g., electrically charged needle tip or nozzle)). The "collector" may also refer elements that attach or connect to the device where electrically charged solution, jet, melt, or gel is deposited onto, where the whole is electrically connected and grounded.

The term "chuck" as used herein refers to a type of clamp used to hold an object with radial symmetry (e.g., a cylinder), and herein may be mechanically and electrically connected via an adaptor to a rotator, in forming a part of a grounded collector. For examples, in drills, a chuck holds the rotating tool or workpiece.

Use of the term "about" is intended to describe values either above or below the stated value in a range of approximately +/- 10%. The preceding ranges are intended to be made clear by context, and no further limitation is implied.

## II. Stents and Methods of Making Stents

Different techniques may be used to prepare polymeric nanofibers, which may be incorporated to form a stent device with a wall thickness ranging from a few microns to a few millimeters, e.g., from about 50 $\mu$m to about 1 mm. Other techniques to cast, solidify, or otherwise produce stent devices with a degradable core may also be used.

*Electrospinning*

An exemplary technique for polymeric nanofibers is electrospinning, which is a simple and adaptable technique that employs electric forces to elongate and reduce the diameter of a viscoelastic polymer jet, allowing for the formation of solid fibers ranging from nanometers to microns in diameter (Li D, Xia Y. Advanced Materials, 16(14):1151-1170 (2004); Bhardwaj N, Kundu SC., Biotechnol Adv. 28(3):325-347 (2010); Ibrahim Z, et al., PCT/US2015/025031).

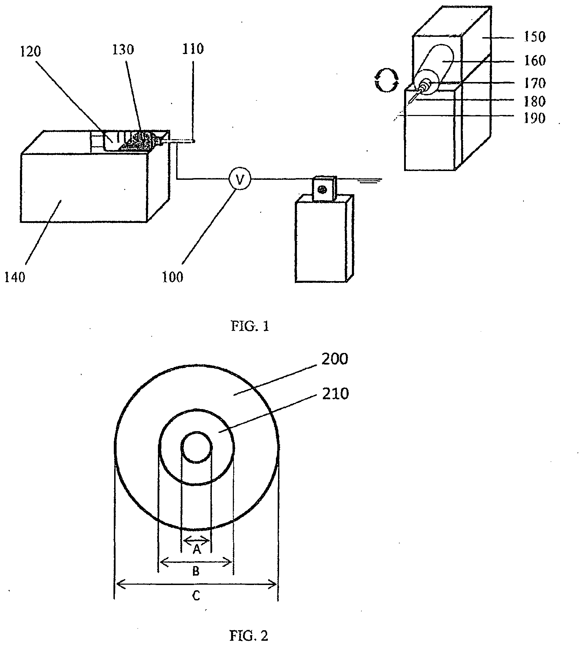

As shown in Figure 1, a 120W regulated high voltage DC power source **100** may be applied to a blunt tip needle **110** on the end of a syringe **120**. This allows for the ejection of electrified polymer solution **130** from the syringe. The flow rate of this solution is controlled by a NE-1000 Programmable Single Syringe Pump **140** mounted onto a plexiglass base atop a motorized stage capable of controlled x- and y-direction motion. The collector consists of a mounted 120V, 1/3hp, 300-3,450rpm speed-control motor **150** (clockwise or counter-clockwise rotation) with a 1.21" shaft. Adaptors **160** are used to connect the shaft of the motor to a 0-3/8" capacity keyless chuck **170** (or to a cylindrical rod in other embodiments). Adaptors contain the motor shaft. An optional blunt tip needle **180** may be directly attached to or enclosed within the drill chuck 170. Alternatively, the template wire may be held within another device such as a template wire **190**. A stand-alone parallel mount allows for the charged polymer jet to deposit fibers between the chuck **170** and the stand-alone parallel mount, where the fibers would later be twisted around the template wire **190**. Later, the deposited fibers are removed from the electrospinning setup with any suitable component such as, without limitation, a blade (knife, scissors, etc.), a wedge, a plate, or any other shaped device that can be utilized to shear or cut the fiber from the collection surface. The template wire **190**, or any cylindrical matter serving as a template to achieve a lumen in the as-formed stents, can be of any length that does not necessarily span from the drill chuck to the stand-alone parallel mount, or it can be long enough to span between the drill chuck and the stand-alone parallel mount.

In some embodiments, grounded rotating collectors or collectors capable of being rotated (e.g., via connection to a motor or pump) of a desired shape or dimensionare used to permit electrically charged polymer solution to deposit as fibers. For example, a syringe needle or a template wire may be grounded to act as a collector for the electrically charged polymer jet.

When the template wire is removed from the deposited fibers, a lumen is left with the fibers forming a stent. Cylindrical stents may be prepared in at least two ways. The first method includes spraying charged polymer jet directly onto a rotating, conductive, cylindrical template of any desired diameter or length for the lumen size of the as-formed stents. The second method includes spraying charged polymer jet so that it deposits between a standstill drill chuck and a standstill parallel stand, where a nonconductive cylindrical template is held in between; and after fibers are deposited, the drill chuck is rotated to uniformly twist the parallel fibers onto the template. The drill chuck can be replaced with any component that can hold the cylindrical template and is connected to a motor to allow for rotation of the cylindrical template or twisting of deposited fibers. Generally, a shaft may be added to connect any object (e.g., cylindrical template) to the motor, and means to attaching a template to an object is known in the art, including, but not limited to, by adhesive means, by fitting inside the larger component, and by being a part of the object.

For example, a biodegradable polymer is dissolved in a solvent used in electrospinning, e.g., hexafluoroisopropanol (HFIP), at a concentration between 0.1 wt% and 50 wt% depending on the polymer's molecular weight and desired viscosity of the polymer solution. This polymer solution is first electrospun onto a template wire, heated past its glass transition temperature for alignment and hardening. Subsequently, a non-degradable polymer is dissolved and electrospun on top of the first polymer surrounding the template wire, and it is also heat treated to improve the mechanical properties. The thickness of each layer can be reproducibly modified by altering the electrospinning duration. Optionally, a thin external coating is applied to the nanofibrous stents, e.g., polyurethane or other polymers at a concentration between 0.01 wt% and 10 wt%. This coating can be applied by pipetting, spraying, or otherwise wetting the surface of the stents, and later snap-frozen and/or lyophilized.

After a jet of polymer solution is collected on the template or substrate, it is heated past the polymer's glass transition temperature to align fibers, change wall patterning, increase strength, and harden the stent.

In addition to the diagramed single needle example, multi-jets from a single needle may be used to obtain higher productivity. A single-jet is initiated from the Taylor cone with the application of electric field, possibly due to two mechanisms: significant discrepancy in electric field distribution and some degree of solution blockage at the needle tip. Alternatively, a curved collector may be used for the formation of multi-jets from multiple Taylor cones in a single needle electrospinning system. When fluid jects interact with large axial electric fields, jet-splitting (i.e. a sequence of secondary jets emanating from the primary jet) may be realized.

In other embodiments, multiple needle electrospinning systems and selection of needle gauge and needle configuration may increase the productivity of electrospinning. Needles can be arranged in linear configuration or two-dimensional configurations such as elliptical, circular, triangular, square and hexagonal. Multiple nozzle heads may be used.

Other techniques may be used to fabricate stent devices made from polymeric materials with a tunable variation on lumen dimensions. Techniques such as meltblowing, bicomponent spinning, forcespinning, and flash-spinning may also be used for preparing polymeric nanofibers, which may be incorporated to form the disclosed at least partially degradable stent whose lumen may enlarge as the polymeric nanofibers of the interior of the stent wall degrade.

Other variations in electrospinning include needleless systems (e.g., the waves of an electrically conductive liquid self-organize on a mesoscopic scale and finally form jets when the applied electric field intensity is above a critical value), bubble electrospinning (e.g., one or several bubbles were formed on the free surface of the solution, when the gas pump is turned on slowly -- the shape of each bubble may change from spherical to conical as the DC voltage is applied -- multiple jets may be ejected from the bubbles when the applied voltage exceeds a threshold value), electroblowing (e.g., simultaneously applying an electrical force and an air-blowing shear force), and others as described by Nayak R, et al., Textile Research Journal, 82(2):129-147 (2011).

*Meltblowing*

In a meltblowing process, a molten polymer is extruded through the orifice of a die. The fibers are formed by the elongation of the polymer streams coming out of the orifice by air-drag and are collected on the surface of a suitable collector in the form of a web. The average fiber diameter mainly depends on the throughput rate, melt viscosity, melt temperature, air temperature and air velocity. Nanofibers can be fabricated by special die designs with a small orifice, reducing the viscosity of the polymeric melt and suitable modification of the meltblowing setup. To reduce or prevent the sudden cooling of the fiber as it leaves the die before the formation of nanofibers, hot air flow may be provided in the same direction of the polymer around the die. The hot air stream flowing along the filaments helps in attenuating them to smaller diameter. The viscosity of polymeric melt can be lowered by increasing the temperature. When the collector is a wire-shaped substrate, the deposited polymer fibers on the substrate may form the disclosed stent when the substrate is removed, resulting in a lumen of the stent.

*Template melt-extrusion*

In template melt-extrusion, molten polymer is forced through the pores of a template (e.g., an anodic aluminum oxide membrane (AAOM)) and then subsequently cooled down to room temperature. A special stainless steel appliance may be designed to support the template, to bear the pressure and to restrict the molten polymer movement along the direction of the pores. The appliance containing the polymer was placed on the hot plate of a compressor (with temperature controlled functions) followed by the forcing of the polymeric melt. Isolated nanofibers may be obtained by the removal of the template (e.g., dissolution with appropriate solvent(s)). When the nanofibers are deposited on a wire-shaped collector, the deposited polymeric nanofibers may form the disclosed stent when the substrate is removed, resulting in a lumen of the stent.

*Flash-spinning*

In the flash-spinning process, a solution of fiber forming polymer in a liquid spin agent is spun into a zone of lower temperature and substantially lower pressure to generate plexi-filamentary film-fibril strands. A spin agent is required for flash-spinning which: 1) should be a non-solvent to the polymer below its normal boiling point, 2) can form a solution with the polymer at high pressure, 3) can form a desired two-phase dispersion with the polymer when the solution pressure is reduced slightly, and 4) should vaporize when the flash is released into a substantially low pressure zone. Flash-spinning is more suitable for difficult to dissolve polymers such as polyolefins and high molecular weight polymers. The spinning temperature should be higher than the melting point of polymer and the boiling point of solvent in order to effect solvent evaporation prior to the collection of the polymer.

*Biocomponent spinning*

Bicomponent spinning is a two-step process that involves spinning two polymers through the spinning die (which forms the bicomponent fiber with island-in-sea (IIS), side-by-side, sheath-core, citrus or segmented-pie structure) and the removal of one polymer.

*Other approaches*

In some embodiments, the disclosed stent devices may be prepared via other methods than electrospinning, such as 3-D printing and dipping in polymer solutions and drying of a cylindrical/wire-shaped template, followed by removal of the template after the polymeric wall of the stent is formed, resulting in a stent device with a wall surrounding a lumen.

**III. Stent or Shunt**

In some embodiments, the disclosed stents include a glaucoma shunt made with non-swelling polymeric nano- and/or micro-fibers, or polymers with a minimal swelling capability. Generally the surface morphology of these polymeric fibers is not porous. Non-swellable polymers generally refer to non-water absorbing and/or water-insoluble polymers herein. In other embodiments, the disclosed stents include a degradable inner lumen within a shunt to modulate outflow of fluid, and intraocular pressure following implantation to a part of the eye.

**A. Stent/shunt Properties**

**1. Dimensions**

The dimension of the lumen can vary depending on the need of operations, generally creating a pressure differential of no less than 5 mmHg for fluid flowing through the device at a physiological flow rate (e.g., averaging 150 $\mu$l/h, may be 50, 100, 180, or 200 $\mu$l/h), thereby avoiding hypotony; and it can be made as the electrospun fibers can deposit to form devices of any dimension. The dimension of the overall/outer diameter and length of the device can vary depending on the ocular anatomy. For glaucoma drainage purposes, the target IOP, both in the short term and long term, is an important factor in determining the dimensions of the device. The Hagen-Poiseuille (HP) equation models the pressure change in relation to fluid dynamics:

$$\Delta P = 8\mu LQ/(\pi r^4) \qquad (1)$$

Equation (1) describes the pressure drop in an incompressible and Newtonian fluid in laminar flow through a long cylindrical pipe of constant cross section. $\Delta P$ is pressure loss, L is length of pipe, $\mu$ is the dynamic viscosity, Q is the volumetric flow rate, and r is the radius. Using previously established values for volumetric flow rate of aqueous humor and fluid viscosity, $\Delta P$ can be modeled for tubes of different lengths and lumen diameters.

For example, a tube with a length of 12 mm and a diameter of 50 $\mu$m would have a venting pressure of 22.81 mm Hg. If the diameter of the tube is increased to 70 $\mu$m, the venting pressure is 5.94 mm Hg (Table 1).

**Table 1. Predicted venting pressure is inversely proportionate to tube lumen diameter.**

| Lumen diameter ($\mu$m) | Length (mm) | Flow rate (mL/hr) | Dynamic Viscosity (cP) | Venting pressure (mm Hg) |
|---|---|---|---|---|
| 50 | 12 | 0.2 | 0.7 | 22.81 |
| 70 | 12 | 0.2 | 0.7 | 5.94 |

For a cylindrical device of a given length, the immediate target IOP drop determines the initial diameter of the lumen of the stent, e.g., before degradation of the degradable inner core; and the target IOP drop in the long-run determines the final diameter of the lumen of the stent, e.g., after the degradable core has completed degraded. The device typically has an initial inner diameter from about 10 $\mu$m to 100 $\mu$m, preferably from 30 to about 80 microns, more preferably from about 40 to about 50 microns. The final lumen diameter of the stent is greater than the initial lumen diameter, as the degradation of the inner core enlarges the lumen. The final lumen diameter (inside diameter) typically is from about 50 $\mu$m to about 200 $\mu$m, more preferably from about 60 $\mu$m to about 80 $\mu$m. In some embodiments, a stent with an initial pressure gradient of ~17 mm Hg and an end pressure gradient of ~5 mm Hg for a tube length of 8 mm has an initial inner diameter of 50 $\mu$m that enlarges to 75 $\mu$m once the biodegradable portion has completely disappeared. To achieve a desired initial pressure gradient or a desired final pressure gradient along the stent, the required initial or final diameters are also dependent on the length of the stent. A 5-mm long stent provides less resistance than an 8-mm long stent, as predicted by the HP equation. Thus, for a 5-mm long stent to achieve the same initial pressure gradient (i.e., IOP loss) of ~17 mm Hg, its required initial inner diameter would be smaller than the required initial inner diameter of 50 $\mu$m for the 8-mm long stent, according to the HP equation. The opposite is true if the stent is longer than 8 mm: the initial inner diameter would have to be larger than 50 $\mu$m to achieve the same initial IOP lowering as an 8-mm long stent. The outer diameter of the device as a stent may be determined by the geometry of the space that the stent will be placed; it may also be determined by the amount of conjunctival scarring in the eye; and it may be determined by the thickness required for stent strength, durability, flexibility and stability depending on the length and polymer used. Cylindrical stent devices as well as stent devices of other shapes such as rectangular, triangular, or others may be varied depending on the ocular anatomy and application specifics.

## 2. Rate of degradation

The rate of the degradation is controlled by selecting the chemical structure, molecular weight, and linear/-branching structure of the degradable polymer, as well as its concentration/density, alignment and degree of interpenetration with the non-degradable fibers in the formed device. In some embodiments, the stent contains a degradable portion, e.g., the inner core in a cylindrical shaped tube, which degrades after implantation into a compartment of the eye in a subject for a period of about 14 days to about 100 days, and preferably over about 2 to about 6 weeks. As this inner core degrades, the lumen diameter enlarges, resulting in a decreased the pressure gradient over time. Once the biodegradable portion of the tube has disappeared, a permanent stent with an inner diameter larger than the initial inner diameter will remain, allowing for long-term pressure reduction.

The degradation, in some embodiments, is primarily driven by hydrolysis of polymers. In other embodiments, the degradation is mediated through temperature changes, pH changes, or catalyzed by enzymes.

## 3. Other properties

The surface properties of the device can be varied depending on the polymer or copolymers to form the stents. The hydrophilicity or hydrophobicity, the surface charge, the wettability, and the biocompatibility can all be varied. In some embodiments, the stent, or when the device is used on its own as a drainage device, is made from materials to which fibroblasts cannot adhere. In some embodiments, the surface charge of the stent is neutral or close to neutral, whereas in other embodiments, it is positively or negatively charged.

The mechanical properties of the stent can also be varied depending on the materials forming the stent. Normally, the strength and hardness of the stent is increased by heat treatments above the glass transition temperature. In some embodiments, the stent is flexible. The stiffness of the stent can also be modified depending on its length, and generally shorter stents are stiffer than longer stents. Generally the device is modified to be strong enough to be implanted into the tissue and for the inner lumen to stay open, i.e., not collapsed, *in vivo.*

The stent devices may be flexible, straight or curved, in order to suit ocular anatomy. The devices may also be tuned to have controlled biological responses (e.g., non- or low adsorption of fouling proteins; enzymatic degradation behavior; cell-adhesion or guidance properties) by selection of polymer compositions, polymeric nanofiber aspect ratios, the porosity of assembled nanofibers in the device, etc. In some embodiments, the porosity of the wall of the stent devices is effective to restrict the penetration of cells to no more than 10%, 20%, 30%, 40%, or 50% of the wall thickness in a time period of 1 week, 2 weeks, 3 weeks, 4 weeks, 2 months, 3 months or 6 months following implantation into a part of the eye. In other embodiments, the porosity of the wall, the composition of the polymeric nanofibers, and their dimensions may be effective to allow the penetration of cells to greater than 10%, 20%, 30%, 40%, or 50% of the wall thickness in a time period of 1 week, 2 weeks, 3 weeks, 4 weeks, 2 months, 3 months or 6 months following implantation, depending on the application. In some embodiments, the selection of polymer composition and the nanofiber diameter are effective to reduce protein adsorption by about 10%, 20%, 30%, 40%, 50%, 60%, 70%, 80%, 90%, or greater when compared to stent devices prepared as a bulk or using building blocks having a larger dimension than the disclosed nanofiber dimensions.

In some embodiments, the polymeric nanofibers forming the stent contain non-swellable polymers or polymers with minimal swelling in an aqueous or physiological medium. In some embodiments, the polymeric nanofibers forming the stent device contain swellable and non-swellable polymers in an aqueous or physiological environment at a mass ratio between about 1:100 and about 100:1, and preferably between about 1:20 and 1:5. Generally, the device includes a lumen surface prepared with degradable polymers that are capable of being absorbed by living mammalian tissue, e.g., through hydrolysis of the hydrolytically instable backbone, over a period of days, week, months, or years and typically the inner core (or the luminal surface) degrades between about 2 and about 100 days.

## B. Biodegradable Polymers

The stents contain one or more biodegradable, biocompatible nanofibers made of suitable polymers. The one or more biodegradable, biocompatible polymers can be homopolymers or copolymers.

Examples of suitable biodegradable, biocompatible polymers include polyhydroxyacids such as poly(lactic acid), poly(glycolic acid), and poly(lactic acid-co-glycolic acids); polyhydroxyalkanoates such as poly-3-hydroxybutyrate or poly-4-hydroxybutyrate; polycaprolactones; poly(orthoesters); polyanhydrides; poly(phosphazenes); poly(hydroxyalkanoates); poly(lactide-co-caprolactones); polycarbonates such as tyrosine polycarbonates; polyamides (including synthetic and natural polyamides), polyesteramides; polyesters; poly(dioxanones); poly(alkylene alkylates); hydrophobic polyethers; polyurethanes; polyetheresters; polyacetals; polycyanoacrylates; polyacrylates; polymethylmethacrylates; polysiloxanes; poly(oxyethylene)/poly(oxypropylene) copolymers; polyketals; polyphosphates; polyhydroxyvalerates; polyalkylene oxalates; polyalkylene succinates; poly(maleic acids); chitosan; poly(hydroxybutyric acid); polyphosphazenes; polyphosphoesters; polysaccharides such as hyaluronic acid; as well as copolymers thereof. Virtually any polymer can be used to prepare fibers via electrospinning; but preferred biocopmpatible polymers are strong, stable, surface-eroding, and soluble in organic solvents, and degradable *in vivo* between about 2 weeks to about 6 weeks, preferably between about 3 weeks to about 4 weeks.

In a preferred embodiment, the biodegradable polymer is poly-(D,L-lactide-co-glycolide) or polyglycolide. In some embodiments, the poly-(D,L-lactide-co-glycolide) contains about 55 to about 80 mole % lactide monomer and about 45 to about 20 mole % glycolide. Alternatively, the poly-(D, L-lactide-co-glycolide) may contain about 65 to about 75 mole % lactide monomer and about 35 to about 25 mole % glycolide. The poly-(D, L-lactide-co-glycolide) can contain terminal acid groups.

The biodegradable, biocompatible polymer can be a polylactic acid polymer or copolymer containing lactide units substituted with alkyl moieties. Examples include, but are not limited to, poly(hexyl-substituted lactide) or poly(dihexyl-substituted lactide).

The molecular weight of the one or more biodegradable, biocompatible polymer can be varied to prepare the degradable portion of the stents having the desired properties, such as drug release rate, for specific applications. The one or more biodegradable, biocompatible polymers can have a molecular weight of about 150 Da to 1 MDa. In certain embodiments, the biodegradable, biocompatible polymers has a molecular weight of between about 1 kDa and about 500kDa, more preferably between about 10kDa and about 150 kDa. For instance, about 40 kDa polycaprolactone, or PLGA (50:50) of up to about 200 kDa may be used.

## C. Non-biodegradable Polymers

The stents also contain one or more generally non-biodegradable fibers made of suitable polymers. Suitable non-degradable polymers include but are not limited to polyalkylene (e.g., polyethylene, polypropylene, or copolymers thereof), polystyrene, polyfluoro alkene (e.g., polyfluoroethylene), polyacrylic acid, polyamides (e.g., nylon, nylon 66, nylon 6 (i.e., polycaprolactam)), polycarbonate, polysulfone, polyurethane, polybutadiene, polybutylene, polyethersulfone, polyetherimide, polyphenylene oxide, polymethylpentene, vinyl halide polymers (e.g., polyvinylchloride), polyvinylidene halides (such as polyvinylidene fluoride and polyvinylidene chloride),, polyphthalamide, polyphenylene sulfide, polyetheretherketone ("PEEK"), polyimide, and polymethylmethacylate. In some embodiments, non-biodegradable polymers include, but are not limited to, ethylene vinyl alcohol copolymers (EVOH), silicones and silicon elastomers, polyolefins, polyisobutylene and ethylene - alpha olefin copolymers, styrene - isobutylene - styrene triblock copolymers, , polyvinyl ethers, polyvinyl methyl ether, poly perfluorinated alkene, polyacrylonitrile, polyvinyl ketones, polyvinyl aromatics, polyvinyl esters, polyvinyl acetates, copolymers of vinyl monomers each other and olefins, ethylene - methyl methacrylate copolymers, acrylonitrile - styrene copolymers, ABC resins and ethylene - vinyl acetate copolymers, alkyd resins, polyoxymethylenes; polyethers, epoxy resins, rayon, rayon - triacetate. In some embodiments, non-degradable polymers include polyethylene terephthalate (PET) or copolymers thereof, polyurethanes, poly(styrene-b-isobutylene-b-styrene) (SIBS; a biostable thermoplastic elastomer) or random copolymers of butyl methacrylate-co-acrylamido-methyl-propane sulfonate (BMA-AMPS).

Non-degradable polymers can include those described in U.S. Patent No. 6,214,901, and by Prasad Shastri in Current Pharmaceutical Biotechnology, 4:331-337 (2003). Other exemplary embodiments of non-degradable polymers include poly(ethylene terephthalate), poly(ethylene), poly(propylene), poly(tetrafluoroethylene), poly(methylmethacrylate), ethylene-co-vinylacetate, poly(dimethylsiloxane), poly(ether-urethanes), poly(sulphone), poly(ethyleneoxide), poly(ethyleneoxide-co-propyleneoxide), poly(vinylalcohol), and copolymers thereof.

The amount of polymer or polymers in the finished stents can vary. In some embodiments, the concentration of the polymer or polymers in the finished stents is from about 75 wt% to about 85% by weight of the finished stents. In some embodiments, the concentration of the polymer or polymers in the finished stents is from about 85 wt% to about 95%, or as high as 100% by weight of the finished stents. Any solvent is mostly evaporated during fiber formation and is almost negligible after proper lyophilization/desiccation/drying methods.

The amount of the non-degradable polymer in a stent may be determined by the geometry and location of the space that the stent will be placed in, the amount of conjunctival scarring in the eye, and the thickness required for stent strength, durability, flexibility, and stability depending on the length and polymer used. The amount of degradable polymer is determined by the desired change in diameter before and after degradation to achieve a desired lowering of IOP.

### D. Exterior coating

After the deposition of degradable and/or non-degradable nanofibers to form the cylindrical structure, an exterior coating may be applied on the surface. The coating materials may be impermeable to water or aqueous fluid in order to prevent leakage, or be able to improve glideability/handleability, modify surface properties (e.g., hydrophilicity/hydrophobicity), and/or to release an agent *in vivo.* The coating materials may be biodegradable or non-degradable.

Exemplary water-impermeable exterior coating on the device includes polymer (non-porous) films made from polyurethane, polyethylenes (such as low density polyethylene) (LDPE), ethylene methyl acrylate (EMA) copolymers and ethylene vinyl acetate copolymers (EVA). A copolyester thermoplastic elastomer such as a copolyetherester elastomer having a randomized hard-soft segment structure is generally permeable to polar molecules such as water but is resistant to penetration by non-polar hydrocarbons such as refrigerant gases. Thermoplastic polyurethane elastomers are basically diisocynates and short chain diols (which form the basis of hard segments) and long chain diols (which form the basis of soft segments). Because the hard and soft segments are incompatible, the thermoplastic urethane elastomers exhibit two-phase structures which in turn cause the formation of domain microstructures. A polyamide thermoplastic elastomer comprising hard and soft segments joined by amide linkages. These thermoplastic polyamide elastomers exhibit properties that depend upon the chemical composition of the hard (polyamide) and the soft (polyether, polyester or polyetherester) blocks as well as their segment lengths. Alternatively, a polymer/polymer composite combining polydimethyl siloxane and polytetrafluoroethylene (PTFE) in an interpenetrating polymer network may be used as a coating; that is, the film is a physical blend of the two polymers rather than a copolymer or new compound.

Other useful coating materials include copolyesters which are very permeable to water vapor and impermeable to liquid water. One example of a suitable material is a copolyester of glycol (1,2-ethanediol) and a mixture of dicarboxylic acids made of the Eastman Company and is known as #14766.

In certain embodiments, specific useful coating materials include high moisture vapor transmission rate grade thermoplastic elastomers such as the Eastman Company's ECDEL® copolyester; DuPont's Hytrel® copolyester; Ato Chem's PEBAX® polyamide copolymer; and Morton International and B. F. Goodrich's polyurethanes. In certain preferred embodiments, an ethylene methyl acrylate copolymer (EMA) an ethylene acrylic acid copolymer (EAA), an ethylene ethyl acrylate copolymer (EEA), an ethylene vinyl acetate copolymer (EVA) or an ethylene methacrylic acid copolymer (EMAA) can be blended with one or more thermoplastic elastomers to improve the draw down and extrudability of the thermoplastic elastomer into a thin coating to overcome any draw down difficulties due to the high elastic nature of the thermoplastic elastomer.

Another suitable coating material comprises a cold water resistant, hot water soluble polyvinyl alcohol coating. The cold water insoluble polyvinyl alcohol film has a high moisture vapor transmission rate and is a very clear and flexible film which is insoluble in cold water.

Still another suitable coating material comprises an alkaline soluble polymer which can be applied by extrusion coating onto a three-dimensional apertured film. The alkaline soluble coating layer is permeable to water vapor and impermeable to liquid water. The alcohol soluble coating layer insoluble in water at normal pH and is soluble in solution with high pH.

In some embodiments, the implant is at least partially coated with at least one polymer film that contains therapeutic, prophylactic or diagnostic agent, the polymer film permitting a delivery of a quantity of the agent to ocular tissues over time.

In another embodiment, polymer coating films may function as substance containing release devices whereby the polymer films may be coupled or secured to the disclosed stent device. The polymer films may be designed to permit the controlled release of the substance at a chosen rate and for a selected duration, which may also be episodic or periodic. Such polymer films may be synthesized such that the substance is bound to the surface or resides within a pore in the film so that the substance is relatively protected from enzymatic attack. The polymer films may also be modified to alter their hydrophilicity, hydrophobicity and vulnerability to platelet adhesion and enzymatic attack. In one embodiment, the polymer film is made of biodegradable material.

### E. Therapeutic, Prophylactic, and Diagnostic Agents

The stents may include one or more therapeutic, prophylactic, and/or diagnostic agents that are encapsulated, blended or conjugated to the polymer of the stents, or encapsulated in, blended /conjugated to sustained release nanoparticle/microparticle formulations that are entrapped in between or conjugated with the fibers of the stents. Agents may be encapsulated in the biodegradable inner core, the exterior coating of the stents, or in the outer slower degrading or non-degrading layer of the tubular device. The agent can be a small molecule agent and/or a biomolecule, such as a protein, peptide, polysaccharide, lipid, nucleic acid, small molecule or combination thereof. Suitable small molecule agents include organic and organometallic compounds. Typical small molecule agents have a molecular weight of less than about 2000 g/mol, preferably less than about 1500 g/mol, more preferably less than about 1200 g/mol, most preferably less than about 1000 g/mol. The small molecule agent can be a hydrophilic, hydrophobic, or amphiphilic compound. Biomolecules typically have a molecular weight of greater than about 2000 g/mol and may be composed of repeat units such as amino acids (peptide, proteins, enzymes, etc.) or nitrogenous base units (nucleic acids).

In preferred embodiments, the agent is an ophthalmic therapeutic, prophylactic or diagnostic agent. Representative therapeutic agents include, but are not limited to, anti-inflammatory drugs, including immunosuppressant agents and anti-allergenic agents, anti-infectious, anti-fibrotic, and anesthetic agents. Some examples of anti-inflammatory drugs include triamcinolone acetonide, fluocinolone acetonide, prednisolone, dexamethasone, loteprednol, fluorometholone. Immune modulating drugs such as: cyclosporine, tacrolimus and rapamycin. Non-steroidal anti-inflammatory drug include ketorolac, nepafenac, and diclofenac. Antiinfectious agents include antiviral agents, antibacterial agents, antiparasitic agents, and anti-fungal agents. Exemplary antibiotics include moxifloxacin, ciprofloxacin, erythromycin, levofloxacin, cefazolin, vancomycin, tigecycline, gentamycin, tobramycin, ceftazidime, ofloxacin, gatifloxacin; antifungals: amphotericin, voriconazole, natamycin.

Agents can include anti-glaucoma agents that lower intraocular pressure (IOP), anti-angiogenesis agents, growth factors, and combinations thereof. Examples of anti-glaucoma agents include mitomycin C, prostaglandin analogs such as travoprost and latanoprost, prostamides such as bimatoprost; beta-adrenergic receptor antagonists such as timolol, betaxolol, levobetaxolol, and carteolol, alpha-2 adrenergic receptor agonists such as brimonidine and apraclonidine, carbonic anhydrase inhibitors such as brinzolamide, acetazolamine, and dorzolamide, miotics (i.e., parasympathomimetics) such as pilocarpine and ecothiopate), seretonergics, muscarinics, and dopaminergic agonists. Suitable agents to include in the device also contain Rho Kinase inhibitors (rho-associated protein kinase inhibitors, or ROCK inhibitors). The Rho kinase (ROCK) isoforms, ROCK1 and ROCK2, were initially discovered as downstream targets of the small GTP-binding protein Rho. Exemplary ROCK inhibitors include, but are not limited to, Fasudil, Ripasudil, RKI-1447, Y-27632, GSK429286A, and Y-30141.

Representative anti-angiogenesis agents include, but are not limited to, antibodies to vascular endothelial growth factor (VEGF) such as bevacizumab (AVASTIN®) and rhuFAb V2 (ranibizumab, LUCENTIS®), and other anti-VEGF compounds including aflibercept (EYLEA®); MACUGEN® (pegaptanim sodium, anti-VEGF aptamer or EYE001) (Eyetech Pharmaceuticals); pigment epithelium derived factor(s) (PEDF); COX-2 inhibitors such as celecoxib (CELEBREX®) and rofecoxib (VIOXX®); interferon alpha; interleukin-12 (IL-12); thalidomide (THALOMID®) and derivatives thereof such as lenalidomide (REVLIMID®); squalamine; endostatin; angiostatin; ribozyme inhibitors such as ANGIOZYME® (Sirna Therapeutics); multifunctional antiangiogenic agents such as NEOVASTAT® (AE-941) (Aeterna Laboratories, Quebec City, Canada); receptor tyrosine kinase (RTK) inhibitors such as sunitinib (SUTENT®); tyrosine kinase inhibitors such as sorafenib (Nexavar®) and erlotinib (Tarceva®); antibodies to the epidermal grown factor receptor such as panitumumab (VECTIBIX®) and cetuximab (ERBITUX®), as well as other anti-angiogenesis agents known in the art.

In some embodiments, the agent is an imidazole antiproliferative agent, a quinoxaline, a phsophonylmethoxyalkyl nucleotide analog, a potassium channel blocker, and/or a synthetic oligonucleotide. In some embodiments, the agent is 5-[1-hydroxy-2-[2-(2-methoxyphenoxy) ethylamino] ethyl]-2-methylbenzenesulfonamide.

In some embodiments, the agent is a guanylate cyclase inhibitor, such as methylene blue, butylated hydroxyanisole, and/or N-methylhydroxylamine. In some embodiments, the agent is 2-(4-methylaminobutoxy) diphenylmethane. In some embodiments, the agent is a combination of apraclonidine and timolol.

In some embodiments, the agent is a cloprostenol analog or a fluprostenol analog. In some embodiments, the agent is a crosslinked carboxy-containing polymer, a sugar, and water. In some embodiments, the agent is a noncorneotoxic serine-threonine kinase inhibitor. In some embodiments, the agent is a nonsteroidal glucocorticoid antagonist. In some embodiments, the agent is a prostaglandin analog or a derivative thereof.

In some cases, the agent is a diagnostic agent imaging or otherwise assessing the eye. Examples of diagnostic agents include paramagnetic molecules, fluorescent compounds, magnetic molecules, and radionuclides, x-ray imaging agents, and contrast media.

The agents may be present in their neutral form, or in the form of a pharmaceutically acceptable salt. In some cases, it may be desirable to prepare a formulation containing a salt of an agent due to one or more of the salt's advantageous physical properties, such as enhanced stability or a desirable solubility or dissolution profile.

Generally, pharmaceutically acceptable salts can be prepared by reaction of the free acid or base forms of an agent with a stoichiometric amount of the appropriate base or acid in water or in an organic solvent, or in a mixture of the two; generally, non-aqueous media like ether, ethyl acetate, ethanol, isopropanol, or acetonitrile are preferred. Pharmaceutically acceptable salts include salts of an agent derived from inorganic acids, organic acids, alkali metal salts, and alkaline earth metal salts as well as salts formed by reaction of the drug with a suitable organic ligand (*e.g.*, quaternary ammonium salts). Lists of suitable salts are found, for example, in Remington's Pharmaceutical Sciences, 20th ed., Lippincott Williams & Wilkins, Baltimore, MD, 2000, p. 704. Examples of ophthalmic drugs sometimes administered in the form of a pharmaceutically acceptable salt include timolol maleate, brimonidine tartrate, and sodium diclofenac.

The agent or agents can be directly dispersed or incorporated into the fibers as particles using common solvent with the polymer, for examples microparticles and/or nanoparticles of drug alone, or microparticles and/or nanoparticles containing a matrix, such as a polymer matrix, in which the agent or agents are encapsulated or otherwise associated with the particles.

The concentration of the drug in the finished stents can vary. In some embodiments, the amount of drug is between about 0.1% and about 30% or even greater by weight, preferably between about 1% and about 20% by weight, more preferably between about 3% and about 20% by weight, most preferably between about 5% and about 20% by weight of the finished stents. Within the inner degradable core, the amount of drug to polymer is between about 0.1% to about 60 wt%. For the exterior coating, the amount of drug is between about 0.1% to about 80%. In preferred embodiments, the agent is an anti-glaucoma agent or an anti-fibrotic/anti-scarring agent, and the concentration of the agent is between about 0.1% to about 50% by weight of the finished stents. The stents releases an effective amount of the antiglaucoma agent to inhibit/prevent the development of glaucoma for at least 2 weeks, 4 weeks, 6 weeks, 8 weeks, 10 weeks, 12 weeks, 16 weeks, or 20 weeks.

### F. Formulations

Pharmaceutical compositions may be for administration to the eye compartment to be treated (e.g., vitreous, subretinal space, subchoroidal space, the episclera, the conjunctiva, the subconjunctiva, the sclera, the anterior chamber, and the cornea and compartments therein, e.g., subepithelial, intrastromal, endothelial), or corneal surface, and can be formulated in unit dosage forms appropriate for each route of administration.

In the preferred embodiment, the stent is place in a trabeculoplasty within the filtration angle of the eye, where the cornea and iris meet.

Representative excipients include solvents, diluents, pH modifying agents, preservatives, antioxidants, suspending agents, wetting agents, viscosity modifiers, tonicity agents, stabilizing agents, and combinations thereof. Suitable pharmaceutically acceptable excipients are preferably selected from materials which are generally recognized as safe (GRAS), and may be administered to an individual without causing undesirable biological side effects or unwanted interactions.

The stent may in some embodiments be coupled with one or more valves to control the flow rate of the fluid, thereby the reduction of IOP.

### IV. Methods of Using

### A. Ophthalmic applications

The device can be used as a tube shunt in the inhibition and treatment of glaucoma. The pathology, as well as the anatomical analysis of the eye and the development of glaucoma, is described in the U.S. Patent No. 7,135,009.

The device can be used as a stent in the lumen of any GDIs to provide occlusion for controlled outflow of fluid to safely reduce intraocular pressure, or simultaneously as drug delivery vehicles, for inhibiting the development or treating the symptoms of glaucoma.

Alternatively, the device can be used to allow outflow of aqueous humor on its own. When the device has exterior coating that prevents leakage, it is preferred in lieu of other GDIs. A method of using the device to drain aqueous humor from the anterior chamber to the intraorbital space includes implanting the device such that one opening of the tubular device is located in the anterior chamber and another opening is located at the intraorbital space, and the tubular portion is located on the sclera. The surgical procedure may include performing a localized peritomy to remove a portion of conjunctiva and tenon capsule, thus creating a trans-scleral tubular channel, for implantation of the tubular device with one end directed towards the anterior chamber and an opposite end directed towards the orbital space.

The stents can be modified by inclusion of a hydrophilic polymer such as PEG or a POLOXAMER® to provide a burst release of an agent, such as an antiglaucoma agent, followed by sustained release over an extended period of time, such as one week, two weeks, 4 weeks, 6 weeks, 8 weeks, 10 weeks, 12 weeks, 14 weeks, 16 weeks, 18 weeks, or 20 weeks.

The stents can be used in a variety of ophthalmic procedures known in the art. For example, they are planted in the eye to divert aqueous humor (the fluid inside the eye) from the inside of the eye to an external reservoir. They can be used in controlling IOP in eyes with previously failed trabeculectomy and in eyes with insufficient conjunctiva because of scarring from prior surgical procedures or injuries. They can also be used in complicated glaucomas, such as uveitic glaucoma, neovascular glaucoma, and pediatric and developmental glaucomas, among others, where they are used independently or in combination with trabeculectomy.

Trabeculectomy is an ophthalmic surgical procedure used in the treatment of glaucoma. Removing part of the eye's trabecular meshwork and adjacent structures allows drainage of aqueous humor from within the eye to underneath the conjunctiva to relieve intraocular pressure. The scleral flap is typically sutured loosely back in place with several sutures. Common complications include blebitis (an isolated bleb infection typically caused by microorganisms such as *Staphylococcus epidermidis*, *Propriobacterium acnes*, or *Staphylococcus aureus*), inflammation, and bleb-associated endophthalmitis.

Endophthalmitis is an inflammation of the ocular cavities and their adjacent structures. It is a possible complication of all intraocular surgeries, particularly cataract surgery, which can result in loss of vision or the eye itself. Endophthalmitis is usually accompanied by severe pain, loss of vision, and redness of the conjunctiva and the underlying episclera. Infectious etiology is the most common and various bacteria and fungi have been isolated as the cause of the endophthalmitis. The patient needs urgent examination by an ophthalmologist and/or vitreo-retina specialist who will usually decide for urgent intervention to provide intravitreal injection of potent antibiotics and also prepare for an urgent pars plana vitrectomy as needed. Enucleation may be required to remove a blind and painful eye.

### B. Other tissue regenerations

In some embodiments, the partially degradable stents are used as conduits in tissue engineering applications such as nerve regeneration and the polymer scaffolds for the development of vasculature.

In other embodiments, the partially degradable stents are used in applications where passive degradation of an internal core is desired.

The present invention will be further understood by reference to the following non-limiting examples.

### Example 1: Electrospinning to generate partially bio-degradable stents ("pressure control shunt") and *in vitro* characterization of degradation in an aqueous environment.

<u>Materials and Methods</u>

Materials used to generate the tubular shaped stents were generally regarded as safe (GRAS) materials approved by the United States Food and Drug Administration (FDA).

*Sequential Electrospinning*

Biodegradable polyglycolide (PGA) was dissolved at 10 wt% in hexafluoroisopropanol (HFIP). Dissolved PGA was electrospun onto the surface of a template steel wire of 50 $\mu$m in diameter, followed by heating above the glass transition temperature of the polymer to improve its mechanical properties.

Next, non-degradable polyethylene terephthalate (PET) dissolved at 10 wt% in HFIP was sprayed on top of the PGA layer, forming the outer wall, which was also treated with heat afterwards.

Finally, the template wire was removed, leaving a hollow, tubular shaped stent made from PGA and PET.

*Degradation of Inner Wall of the Tube*

The partially degradable, PGA-PET tubes were submerged in phosphate buffered saline (PBS) for 30 days at room temperature, and examined under scanning electron microscopy (SEM). For ease of visualization of the degraded volume, the template wire was not removed from the center of these tubes.

*Scanning Electron Microscopy (SEM)*

The tubes were examined under SEM for their morphology and cross sections before and after submersion in PBS.

Results

As shown in Figure 1, the configuration for electrospinning employed electric forces to elongate and to reduce the diameter of a viscoelastic polymer jet, allowing for the formation of solid fibers.

The inner wall **210** of this tube was composed of biodegradable polymer while the outer wall **200** was non-degradable and permanent (Figure 2). In this example, A was 50 $\mu$m, B was 75 $\mu$m, and C was 350 $\mu$m.

SEM confirmed these tubes were cylindrical, uniform, and composed of nano- and micro-fibers. When the template wire was removed, the tube was hollow in the center under SEM. With the template wire still in place, the partially degradable tube after 30-day submersion in PBS had some enlarged void space between the template wire and the inner surface of the tubes, compared to tubes before submersion in PBS. This void space was believed to be caused by the degradation of PGA fibers.

Agents could be loaded into the stents (e.g., in the biodegradable, inner wall), providing a drug-delivery functionality independent of and/or in association with the degradation of the inner wall of the stents.

**Example 2: Experimental measurements and mathematical modeling of the pressure change of flows through stents**

Materials and Methods

PBS was flowed at different flow rates at room temperature over the period of one day through the stents made as described in Example 1, and the actual pressure change (i.e., pressure difference between inlet end and outlet end of the stent) of the fluid was measured. It was unlikely that core degradation was observed or it would affect the result of pressure change within the relatively short test period. The measured values were compared with the predicted pressure change according to the Hagen-Poiseuille (HP) equation.

Alternatively, the pressure changes related to a wide range of variables (e.g., lengths of stents, radii of stents, and flow rates of a fluid) were mathematically modeled.

Results

As shown in Table 2, the flow of PBS through these stents obeyed the HP equation.

**Table 2. Predicted and actual pressure change with flow of PBS at different flow rates through manufactured stent.**

| Diameter ($\mu$m) | Length (mm) | Flow Rate (mL/hr) | Viscosity (cP) | Predicted pressure change (mm Hg) | Actual pressure change (mm Hg) |
|---|---|---|---|---|---|
| 50 | 4.5 | 0.1 | 1 | 6.23 | 6 |
| 50 | 4.5 | 0.2 | 1 | 12.1 | 11 |
| 50 | 4.5 | 0.4 | 1 | 24.2 | 21 |

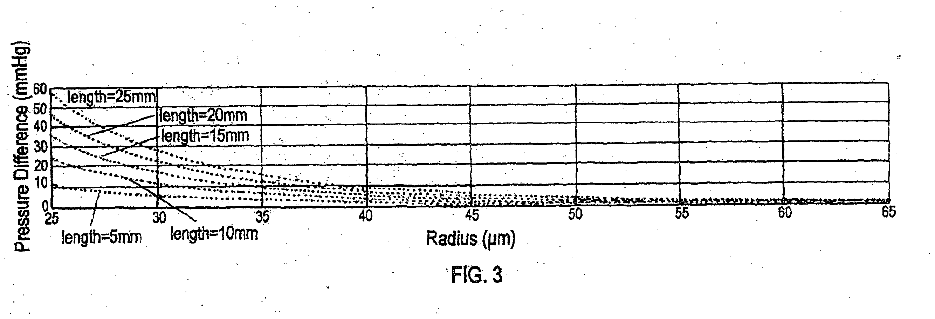

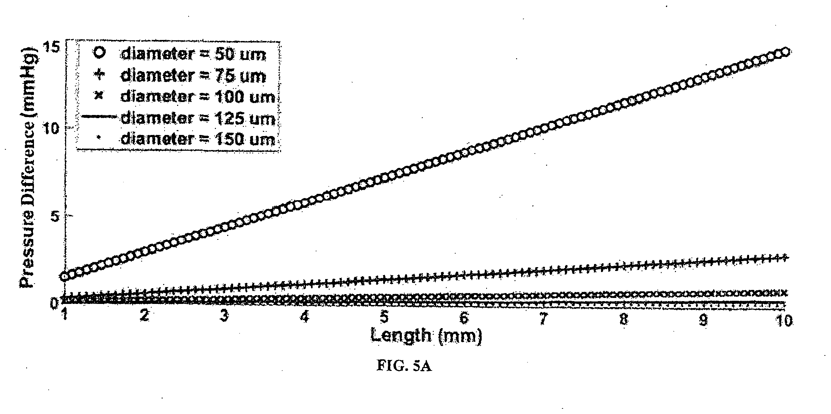

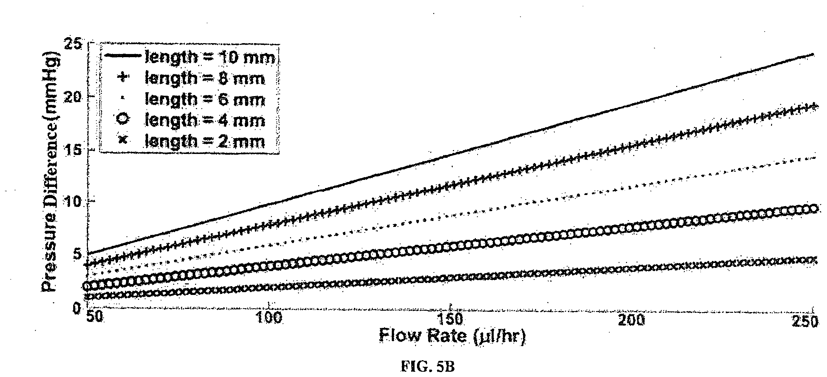

As shown in Figures 3, 5A, and 5B, the radius (r, where 2r = diameter) of the tube lumen had a larger influence on the pressure gradient than tube length, aqueous flow rate, or dynamic viscosity. This was based on the HP equation wherein the pressure gradient is proportional to $1/r^4$.

It was determined via the mathematical modeling of an 8-mm long stent that the stent would need to have an initial inner diameter (i.e., lumen diameter) of 50 $\mu$m that would enlarge to 75 $\mu$m after degradation of inner portion, in order to have an initial pressure gradient of ~17 mmHg along the stent lumen before enlargement of lumen and an end pressure gradient of ~5 mmHg after enlargement of lumen due to degradation of inner wall of the stent. A stent with these design parameters should offer immediate post-operative control on the reduction of IOP as well as drainage of excess fluid pressure in the long term. After insertion into an eye, this stent's inner diameter should gradually enlarge over 2-6 weeks until the inner wall is completely degraded. The IOP at which water flows through the tube was expected to decrease with the enlargement of the stent's inner diameter. Once the biodegradable portion of the tube disappears, in this embodiment with a non-degradable outer wall for the stent, a permanent stent with a larger inner diameter remains, allowing for long-term pressure reduction in a patient.

By increasing the diameter of the tube lumen over time, these partially degradable stents should minimize early post-operative hypotony (e.g., due to fluid outflow through cavity between wounded tissue and the implanted stent) by creating an elevated initial venting resistance (i.e., an elevated differential pressure initially when the lumen diameter was small). They should also minimize conjunctival exposure to aqueous humor in the early post-operative period and provide limited IOP reduction via the stent lumen in the early post-operative period, and maximize the potential for sustained IOP lowering by creating a low venting pressure after disappearance of the biodegradable, inner wall.

**Example 3: Exterior coating of stents to prevent leakage**

Materials and Methods

Polyurethane was dissolved in glacial acetic acid at 0.2 wt%, and the solution was pipetted onto stents as a thin coating and then snap-frozen. The externally coated stents were examined under SEM.

Results

This coating appeared to make the tube impermeable and prevent leakage. It could also be loaded with agents to provide for drug delivery functionality. SEM confirmed the surface smoothness of polyurethane-coated stents was different from that of uncoated stents.

**Example 4: Reduction of IOP in rabbits via non-absorbable stent device**

Materials & Methods

*Preparation of Electrospun Shunt*

Polyethylene terephthalate (PET) shunts were manufactured through electrospinning by utilizing a grounded collector including a drill chuck 170 (obtained from McMaster-Carr, Elmhurst, IL) and parallel collector stand positioned perpendicular to the polymer jet. Stainless steel wire 190 with a diameter of 50 $\mu$m (McMaster-Carr) was inserted through a 25 G, 3.81 cm long, blunt tip needle **180** (Nordson EFD, Westlake, OH) which was then placed into the head of the drill chuck **170.** PET was chosen because it was generally regarded as safe, had great biostability and a well-understood fibrotic response, and has been used in medical devices for over 50 years (Khan W., et al., Focal Controlled Drug Delivery, Boston, MA: Springer US; 2014:33-59). PET (from Nanofiber Solutions, Columbus, OH) was dissolved in 1,1,1,3,3,3-hexafluoro-2-propanol (HFIP; Sigma Aldrich, St. Louis, MO) at 10 wt% by stirring at 45 $^0$C for 24 h. The polymer solution was then electrospun onto the wire (positioned perpendicular to the polymer jet) rotating clockwise at 175 rpm by applying 15 kV of voltage from a power supply **100** (Glassman High Voltage, High Bridge, NJ) to a 20 G blunt tip needle (Nordson EFD) with the polymer solution flowing at a controlled rate of 1.5 mL/h using a programmable syringe pump **140** (New Era Pump Systems, Farmingdale, NY). The shunt was then heated in an oven (Sheldon Manufacturing, Cornelius, OR) for 24 h at 100 $^0$C prior to cooling to room temperature (RT).

*Shunt characterization*

Outer shunt diameter was measured via optical microscopy using an Eclipse TS100 (Nikon Instruments, Melville, NY). Inner lumen diameter and shunt morphology were examined via scanning electron microscopy (SEM) at 1 kV with a LEO Field Emission SEM (Zeiss, Oberkochen, Germany) following desiccation for at least 24 h and sputter coating with 10 nm of Au/Pd. Imaging was conducted prior to removal of the template wire, after removal of the wire, and following *in vitro* fluid flow studies.

*Evaluation of in vitro Fluid Flow through Shunt*

PET Shunts were cut to 5, 6, or 7 mm in length (n=3 for each length), after which the template wire was removed. The shunt was then connected in circuit to a syringe pump and manometer (NETECH, Farmingdale, NY) via the 25 G needle. All flow studies were conducted at RT. 1$\times$ Dulbecco's Phosphate Buffered Saline (PBS, ATCC, Manassas, VA) was pumped through the shunt at 50, 100, and 200 $\mu$L/h. Prior to flow studies, PBS was pumped through a 25 G needle at the specified flow rate and the resulting pressure measurement was subtracted from the pressure observed with attachment of the PET shunt. Pressure measurements were recorded at least 30 min after a change in flow rate and only if the pressure remained constant for more than 5 min. Long-term flow studies (n=3) were conducted by flowing PBS through a 6 mm shunt at 150 $\mu$L/h for 7 days. Theoretical pressure estimates were obtained from the HPE with tube diameter of 50 $\mu$m and 25 $^0$C PBS viscosity of 0.9 cP. (Lavoisier A, et al., MAbs, 7(1):77-83 (2015))

*In vivo Performance and IOP Measurement*

All animals involved in these studies were cared for in accordance with protocols approved by the Johns Hopkins University Animal Care and Use Committee. Protocols are also in accordance with the ARVO Statement for the Use of Animals in Ophthalmic and Vision Research. New Zealand White rabbits (Robinson Services, Mocksville, NC) were sedated by subcutaneous injection of Ketamine:Xylazine (75:5 mg/kg, Sigma Aldrich). A drop of 0.5% 134 proparacaine hydrochloride ophthalmic solution (Bausch & Lomb, Tampa, FL) followed by a 135 drop of 5% betadine solution (Alcon, Fort Worth, TX) was administered to the operative eye. After placement of an 8-0 silk, stay suture (Ethicon, Somerville, NJ) in the superotemporal limbus, a two-clock hour, fornix-based peritomy was created superotemporally, and the conjunctiva was dissected posteriorly. A 25 G needle (Fisher Scientific, Waltham, MA) was used to create a 2 mm long scleral tunnel prior to entering the anterior chamber. The shunt was gently guided through this tunnel. Once the shunt was in position, the template wire was removed and a 10-0 nylon cross-stitch (Ethicon) was placed at the site of tube entry to prevent fluid leakage around the tube. Balanced saline solution (BSS, Alcon) was irrigated in the anterior chamber using a 30 G needle (Fisher Scientific) to validate shunt patency. The conjunctiva was approximated to the limbus using two 10-0 nylon sutures and, again, BSS was irrigated into the anterior chamber to ensure that a water-tight bleb had formed. The stay suture was then removed and topical antibiotic ointment was administered to the eye. In this manner, 3 open lumen and 3 closed lumen (containing the template wire) PET shunts were implanted. All shunts were 6 mm in length. Rabbits were monitored daily for signs of infection, inflammation, or irritation, and were monitored periodically through the duration of the experiment. IOP was measured and calibrated as previously reported. (Fu J, et al., Molecular Pharmaceutics, 13(9):2987-2995 (2016)) Briefly, IOP was measured at 1, 4, 7, 11, 14, 151 21, and 27 days using a TonoVet (iCare, Vantaa, Finland) rebound tonometer in awake, restrained rabbits without topical anesthesia.

*Assessment of in vivo Biocompatibility*

Open and closed PET shunts (n = 3, each) were implanted into New Zealand White rabbits via an ab externo procedure, as described. After monitoring for 27 days, rabbits were euthanized and eyes enucleated, fixed in formalin (Sigma Aldrich), embedded in paraffin, cross-sectioned, and stained with hematoxylin and eosin for histological evaluation.

*Simulation of Fluid Flow through Stunt*

Modeling of pressure differential through the shunt was accomplished through use of MATLAB$^{®}$ (Mathworks, Natick, MA) and according to the HPE equation by varying flow rate, shunt diameter, and shunt length. The viscosity (0.72 cP) and average flow rate (150 $\mu$L/h) of human aqueous humor at physiological conditions were input for the values of $\mu$ and Q, respectively.(Sheybani A, et al., Invest Ophthalmol Vis Sci., 56(8):4789-4795 (2015))

*Statistical Analysis*

Results are presented as mean $\pm$ SE. IOP data were analyzed using a Student's t-test, and differences between parameters were considered statistically significant for p values < 0.05.

<u>Results</u>

*Simulated Pressure Change*

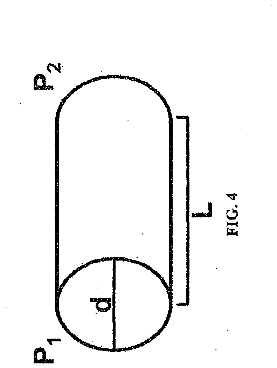

Under physiological conditions, aqueous humor is an incompressible, Newtonian fluid (Sheybani A, et al., Invest Ophthalmol Vis Sci., 56(8):4789-4795 (2015)). Thus, by utilizing the HPE and physiological values of aqueous humor viscosity and flow rate, the dimensions of a non-valved, cylindrical shunt were determined which were capable of providing IOP reduction while also providing sufficient resistance to flow to prevent hypotony (Fig. 4 showing the length, *L*, and the lumen diameter, *d*, of a shunt, and the pressures at entrance and outlet of the shunt, $P_1$ and $P_2$, depending on the direction of flow). Through variation of shunt diameters with a flow rate held constant at 150 $\mu$L/h, simulations revealed that a cylindrical shunt with inner diameter $\geq$ 75 $\mu$m would lead to hypotony at any physiologically relevant shunt length (Fig. 5A). However, a shunt with a 50 $\mu$m inner diameter and greater than 4 mm in length may provide sufficient resistance to flow to maintain IOP above 5 mmHg. In order to achieve significant IOP reduction while simultaneously avoiding hypotony, 5-6 mm long shunts may be desirable. Through variation of shunt lengths with an inner diameter held constant at 50 $\mu$m, the simulation confirmed that a 6 mm long shunt was likely to avoid IOPs associated with hypotony (Fig. 5B), even if the aqueous humor flow rate were to fall below its physiological average of 150 $\mu$L/h.

*Shunt characterizations*

Based on the simulation of aqueous humor flow through a cylindrical shunt at physiological conditions, non-absorbable, nanostructured shunts were prepared with a cross-sectional dimension of a lumen size of 50 $\mu$m in diameter and an overall shunt cross-sectional diameter (outer diameter) of about 400 $\mu$m. A dense and uniform coating of PET nanofibers were deposited onto the template wire with a low wall porosity (as confirmed under SEM) following heat treatment. In order to achieve high strength and maintain lumen integrity *in vivo,* shunts were manufactured with a wall thickness of 354 $\pm$ 15 $\mu$m, and composed of nanofibers, which were believed to possess a higher tensile strength than larger diameter electrospun fibers such as microfibers (Wong S-C, et al., Polymer, 49(21):4713-4722 (2008) showing a decrease in fiber diameter leads to an increase in fiber tensile strength) 5, 6, and 7 mm long shunts were manufactured with an internal lumen diameter of 50 $\mu$m (formed due to removal of the 50 $\mu$m template wire). Prior to *in vitro* flow studies, the shunt was cut to the appropriate size, and the template wire was removed from the inner lumen.

*In vitro performance and flow through shunt*

The manufactured PET shunts were evaluated *in vitro* to determine if they were leak-proof and durable, as well as providing the resistance to flow predicted by the Hagen-Poiseuille equation (HPE). PBS was pumped through 6 mm long PET shunts at a flow rate of 150 $\mu$L/h for a period of one week to evaluate shunt integrity and durability. SEM imaging confirmed the lumen of the shunt remained open and unobstructed maintaining its size after one week of continuous flow. The inner surface of the shunt was composed of aligned nanofibers showing no degradation or breaking. Moreover, leakage of PBS through the shunt wall was not observed through the duration of the study.

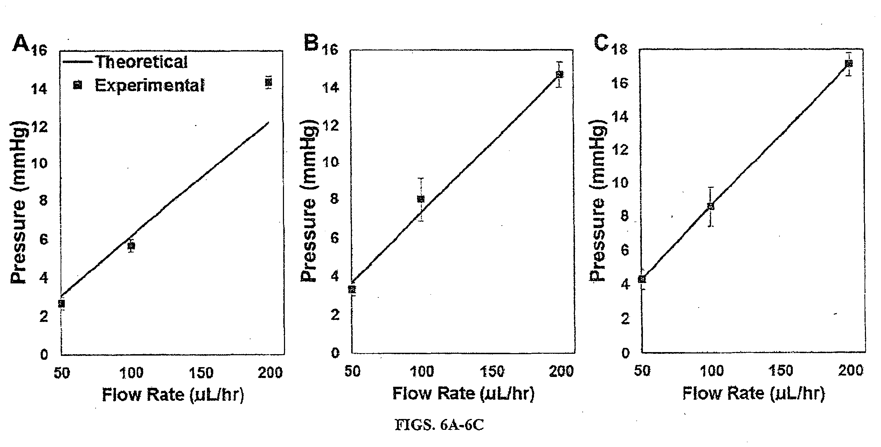

Pressure was also monitored as PBS was pumped through 5, 6, and 7 mm long shunts at physiologically relevant flow rates of 50, 100, and 200 $\mu$L/h. The fluid flow studies demonstrated resistance to flow increased as the flow rate increased and as the shunt length increased (figures 6A-6C). At each shunt length, there was a strong correlation between the experimental measurements and theoretical pressure values predicted by the HPE. This correlation became stronger as shunt length and thereby the entrance length (i.e., the length of the entry region, the distance a flow travels before the flow becomes fully developed) increased, such that allowing the fluid to transition into laminar flow in the main portion of the shunt. The percent difference between the slope of the trend line calculated using linear regression of experimental data and the slope of theoretical values was 22%, 8%, and 0%, respectively, for 5, 6, and 7 mm long shunts.

*IOP Reduction*

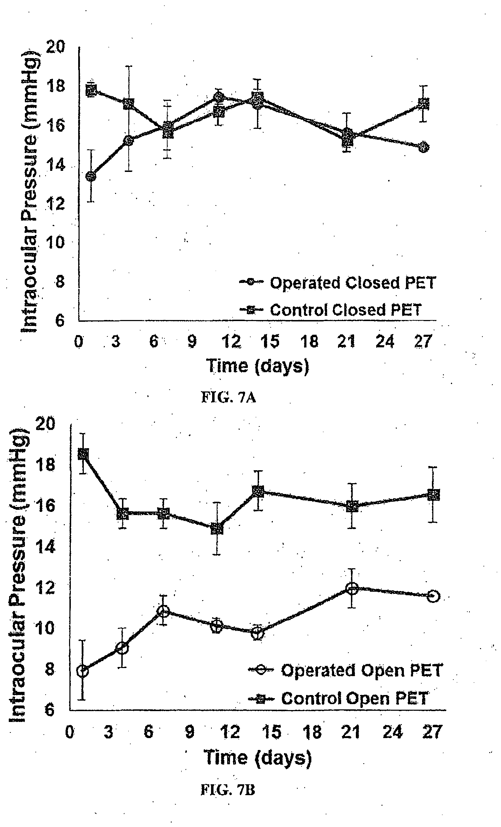

6 mm long PET shunts with either an open or closed lumen were implanted in normotensive rabbit eyes for 27 days via an *ab externo* procedure designed to vent aqueous humor via subconjunctival outflow. Procedure time ranged from 8-12 min for both types of shunts, and no complications were observed during the procedure or during the immediate post-operative period. The lowest average IOP for rabbits with either open or closed PET shunts was observed 24 h post-operatively, indicating that the procedure itself may have contributed to IOP reduction in the immediate post-operative period (figures 7A and 7B). However, hypotony and flattening of the anterior chamber were not observed in the immediate, early, intermediate, or late post-operative period. The IOP of operated rabbit eyes containing closed PET shunts was not significantly different from that of the respective control contralateral eye. The decrease in IOP ranged from -4% to 25% on individual days following implantation, with the maximum decrease observed immediately post-operatively. Operated rabbit eyes containing open PET shunts demonstrated a statistically significant decrease in IOP in comparison to the control contralateral eye. IOP reduction ranged from 24% to 57% on individual days following implantation. The IOP of operated eyes containing open PET shunts was also significantly lower than that of operated eyes containing closed PET shunts. Therefore, nano-structured, open-lumen shunts significantly decreased IOP in rabbit eyes in comparison to closed-lumen shunts and non-operated contralateral eyes.

*In vivo Biocompatibility*

Nanostructured PET shunts were well-tolerated by rabbit eyes. Implanted shunts did not migrate into the anterior chamber during the course of the study, and there were no gross signs of infection, hyphema, or inflammation with either open or closed shunt implantation. Histological analysis of control subconjunctival tissue and tissue surrounding implanted shunts revealed the migration of cells to the periphery, and into the wall of PET shunts. Neovascularization was not observed in the immediate vicinity of either open or closed PET shunts. Notably, tissue sections also reveal that open PET shunts maintained their original inner and outer diameters for at least 27 days following *in vivo* implantation.

*Discussion*

Non-absorbable PET shunts have been demonstrated to be strong, leak-proof, and to mirror the fluid dynamics expected by the Hagen-Poiseuille Law at varying lengths and flow rates.

In the natural environment, cells interact with nanoscale architecture/cues within the surrounding extracellular matrix. These cues can affect cell migration, morphology, orientation, and even gene regulation. Kam and coworkers have demonstrated that nanoscale features with high aspect ratios decrease expression of several growth factors associated with fibrosis (Kam KR, et al., Tissue Eng Part A, 20(1-2): 130-138 (2014)). The methods used to manufacture marketed drainage devices, such as the iStent[®], MicroShunt[®], or XEN Gel Stent were limited in their capacity to achieve anti-fouling effects through manufacturing alone (Richter GM, et al., Clin Ophthalmol., 10:189-206 (2016)). Protein adsorption has been implicated in glaucoma device fouling and can lead to infection, increase in IOP, or device failure (Harake RS, et al., Ann Biomed Eng., 43(10):2394-2405). The exemplary electrospun nanofibers and shunt made therefrom provided a substantial advantage in that it was likely able to decrease expression of soluble factors associated with fibrosis by surrounding cells, reduce protein adsorption, or a combination of both, for an anti-fouling property. Although porosity of nanofibrous devices may be vulnerable to cell and/or tissue ingrowth in the long run (Khan W, et al., Focal Controlled Drug Delivery, Boston, MA: Springer US; 2014:33-59), the exemplified study found only cell migration to the periphery of the shunts.

Although the commercially available XEN Gel Stent was also designed based on the Hagen-Poiseuille equation (HPE), it is vulnerable to hypotony and anterior chamber shallowing in the immediate post-operative period at inner diameter dimensions of 63 or 140 $\mu$m (Sheybani A, et al., J Cataract Refract Surg. 2015;41(9):1905-1909). Due to the small lumen of the XEN Gel Stent with a 45 $\mu$m inner diameter, fouling could theoretically affect its function over the long-term (Sheybani A, et al., J Glaucoma, 25(7):e691-6 (2016)). Venting resistance is inversely proportional to $r^4$, thus, small variations in shunt diameter can have a considerable effect on venting IOP. Moreover, the HPE may have limitations in predict the performance of shunts *in vivo*, when the shunts are curved to suit ocular anatomy. Additionally, *in vitro* fluid flow studies demonstrated that shorter PET shunts demonstrated a weaker correlation with the HPE. This may be due to the entrance length required to transition into stable, laminar flow, and is another factor to consider in the HPE-driven design of glaucoma shunts (Sharp KV, et al., Experiments in Fluid, 36(5):741-747 (2004)).

The exemplified open PET shunts maintained their inner diameter and significantly decreased IOP for at least 27 days in comparison to the non-operated contralateral eye. This is in stark contrast to previously conducted trabeculectomy and drainage implant procedures in healthy New Zealand White rabbits. Previously, trabeculectomy procedures in these rabbits fail within 7 days without the use of an antifibrotic or antimetabolite agent (Echavez M, et al., Philipp J Ophthalmol.37(1):45-51 (2012)). In a prospective, non-randomized study of fourteen patients, the MicroShunt[®] demonstrated a 49.8% reduction in IOP in conjunction mitomycin C after 3 years; however, prior studies in New Zealand White rabbits demonstrated no significant change in IOP in comparison to non-operated eyes or to implantation of silicone tubing with an inner diameter of 300 $\mu$m (Acosta AC, et al., Archives of Ophthalmology, 124(12):1742-1749 (2006); Batlle JF, et al., J. of Glaucoma, et al.,25(2):e58-e65 (2016)). This indicates that open PET shunt even in the absence of antifibrotic or antimetabolite agent may be able to vent significantly more aqueous humor in comparison to previous drainage systems.

**Example 5. Fully absorbable, drug-eluting shunt**

Fully absorbable, drug-eluting shunts have been designed to provide outflow while also releasing a drug to modify the conventional outflow pathway to restore natural, physiologic flow.

Poly-L-lactic acid (PLLA) was used as an absorbable polymer to prepare nanofibers that deposited and aligned to form the stent device. Ethacrynic acid (ETA) was included in the polymeric nanofiber, forming shunts PLLA/ETA. ETA was included in the polymeric formulation at 10 wt% in respect to PLLA. This drug has been studied to affect the cytoskeleton of outflow-pathway cells. Cells in the pathway have been shown to change shape (e.g., cytoskeletal contract) and thereby allow more fluid to flow between them. Cell biologists have discovered that rho kinase was one of the master cytoskeletal enzymes. Interestingly, inhibiting the rho kinase enzyme also greatly increased outflow and relaxed the cells.

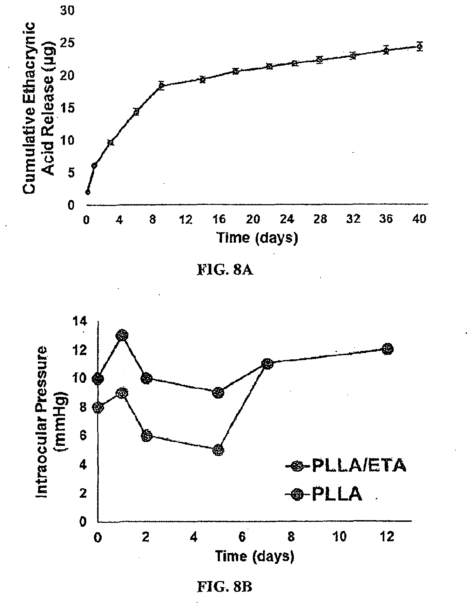

Figure 8A shows the cumulative release ethacrynic acid from 1 cm length PLLA/ETA shunt *in vitro* in PBS at 37 °C. Release media was collected periodically over a period of 40 days, and ethacrynic acid content in release media was detected via high performance liquid chromatography.

Figure 8B shows the intraocular pressure after implantation of PLLA-ETA shunts in comparison to PLLA shunts without ETA. Shunts were implanted into the anterior chamber of normotensive New Zealand White rabbits. The shunts were biocompatible and did not cause infection or significant inflammation or irritation. Eyes receiving implantation of the PLLA/ETA shunt demonstrated decreased intraocular pressure for a period of one week in comparison to eyes receiving implantation of the PLLA shunt. Overall, this demonstrates drug delivery via glaucoma shunts in order to decrease intraocular pressure and/or remodel the outflow pathways.

**Claims**

1. An implantable device comprising a wall surrounding a lumen, wherein the wall comprises

   a first, degradable polymer, forming the luminal surface of the wall,
   and a second, slower degradable or non-degradable polymer, compared to the first degradable polymer, forming the exterior surface of the wall,
   wherein the wall is formed of polymeric nanofibers,
   wherein the nanofibers are aligned and hardened by heating at a temperature greater than the glass transition temperature of the polymer to increase mechanical strength and flexibility compared to the polymer that is not aligned and hardened, and
   wherein the lumen enlarges over time as the first degradable polymer degrades.

2. The device of claim 1, wherein the device comprises a coating on the exterior surface of the wall.

3. The device of claim 1 or claim 2, wherein the device further comprises one or more therapeutic, prophylactic, or diagnostic agent.

4. The device of any one of claims 1 to 3, wherein the nanofibers are made by electrospinning.

5. The device of any one of claims 1 to 3, wherein the first polymer degrades over a time period between about 2 days and about 100 days *in vivo*.

6. The device of any one of claims 1 to 3, wherein the lumen has an averaged overall diameter between 40 $\mu$m +/- 10% and 100 $\mu$m +/- 10%, preferably between 50 $\mu$m +/- 10% and 80 $\mu$m +/- 10%, and more preferably of 50 $\mu$m +/- 10%, before the first polymer degrades.

7. The device of any one of claims 1 to 3, wherein the lumen has an averaged overall diameter between 50 $\mu$m +/- 10% and 120 $\mu$m +/- 10%, preferably between 60 $\mu$m +/- 10% and 90 $\mu$m +/- 10%, after the first polymer degrades.

8. The device of any one of claims 1 to 7, wherein:

   (i) the first degradable polymer, the second slower degradable polymer, or both comprises polyglycolide, poly(lactic acid), poly(caprolactone), chitosan, poly(hydroxybutyric acid), polyanhydrides, polyesters, polyphosphazenes, polyphosphoesters, polydioxanone, poly(lactic-co-glycolic acid), or a blend or copolymers thereof; or
   (ii) the non-degradable polymer comprises poly(ethylene terephthalate), poly(ethylene), poly(propylene), poly(tetrafluoroethylene), poly(methylmethacrylate), ethylene-co-vinylacetate, poly(dimethylsiloxane), poly(ether-urethanes), poly(sulphone), poly(ethyleneoxide), poly(ethyleneoxide-co-propyleneoxide), poly(vinylalcohol), or copolymers thereof.

9. The device of claim 2, wherein the exterior coating comprises polyurethane, silicone, polydimethyl siloxane, polytetrafluoroethylene, or copolymers thereof.

10. The device of claim 1, wherein the device has a tubular structure with at least two ends for passage of fluid through the lumen of the device; optionally wherein a fluid pressure difference between both ends of the device changes from between 0 mm Hg and 25 mm Hg +/- 10% to between 0 mm Hg and less than 15 mm Hg as the lumen enlarges.

11. The device of any one of claims 1 to 10, wherein the device has a length between 0.5 mm +/- 10% and 30 mm +/- 10%, preferably between 1 mm +/- 10% and 20 mm +/- 10%, and more preferably between 3 mm +/- 10% and 10 mm +/- 10%.

12. The device of any one of claims 1 to 11, wherein the wall has a thickness between 50 $\mu$m and 1 mm, preferably between 100 $\mu$m +/- 10% and 500 $\mu$m +/- 10%, optionally wherein the first polymer forms a thickness of the wall between 10 $\mu$m +/- 10% and 80 $\mu$m +/- 10% before degradation.

13. The device of any one of claims 1 to 12, wherein the first polymer and the second polymer have a mass ratio between about 1:50 and about 10:1.

# EP 3 426 315 B1

**14.** The device of claim 3, wherein the one or more agents are encapsulated, associated, or bonded in the device at a weight percentage of between 0,09% and 66%.

**15.** A device according to claim 4, wherein the device is prepared by sequentially extruding at least two electrically charged polymer solutions, each optionally comprising a therapeutic, prophylactic, or diagnostic agent, toward a grounded collector,

> wherein the two polymer solutions form polymeric fibers around the collector, and a first polymer solution comprises a degradable polymer and a second polymer solution comprises a slower degradable polymer, compared to the first polymer, or non-degradable polymer,
> wherein removing the polymeric fibers from the collector results in the wall of the device surrounding a lumen, and wherein the lumen enlarges over time as the degradable polymeric fibers degrade.

**16.** A device as defined in any one of Claims 1 to 15 for use in a method of controlling intraocular pressure reduction in a patient in need thereof, comprising diverting aqueous humor from inside an eye through said device, wherein the device is implanted into a part of the eye of the patient; optionally wherein the patient has glaucoma or ocular hypertension.

**17.** A device as defined in any one of Claims 1 to 15 for use in a method of healing, fixing, replacing, or connecting tissue in a patient in need thereof, comprising implanting said device in a part of the body of the patient; optionally wherein the device is implanted to replace or connect nerve tissue, vasculature, or compartments of eye.


## Patentansprüche

**1.** Implantierbare Vorrichtung, die eine Wand umfasst, die ein Lumen umgibt, wobei die Wand Folgendes umfasst

> ein erstes, abbaubares Polymer, das die Lumenoberfläche der Wand bildet,
> und ein zweites, langsamer abbaubares oder nicht abbaubares Polymer, das im Gegensatz zum ersten, abbaubaren Polymer die Außenoberfläche der Wand bildet,
> wobei die Wand aus Polymer-Nanofasern besteht,
> wobei die Nanofasern durch Erhitzen auf eine Temperatur über der Glastemperatur des Polymers ausgerichtet und gehärtet sind, um die mechanische Festigkeit und Flexibilität im Vergleich zu einem Polymer, das nicht ausgerichtet und gehärtet ist, zu erhöhen, und
> wobei das Lumen im Laufe der Zeit an Größe zunimmt, wenn das erste, abbaubare Polymer abgebaut wird.

**2.** Vorrichtung nach Anspruch 1, wobei die Vorrichtung eine Beschichtung auf der Außenoberfläche der Wand umfasst.

**3.** Vorrichtung nach Anspruch 1 oder Anspruch 2, wobei die Vorrichtung außerdem ein oder mehrere therapeutische, prophylaktische oder diagnostische Mittel umfasst.

**4.** Vorrichtung nach einem der Ansprüche 1 bis 3, wobei die Nanofasern durch Elektrospinnen hergestellt sind.

**5.** Vorrichtung nach einem der Ansprüche 1 bis 3, wobei das erste Polymer *in vivo* über einen Zeitraum zwischen etwa 2 Tagen und etwa 100 Tagen abgebaut wird.

**6.** Vorrichtung nach einem der Ansprüche 1 bis 3, wobei das Lumen einen durchschnittlichen Gesamtdurchmesser zwischen 40 $\mu$m +/- 10 % und 100 $\mu$m +/- 10 %, vorzugsweise zwischen 50 $\mu$m +/- 10 % und 80 $\mu$m +/- 10 % und noch bevorzugter von 50 $\mu$m +/- 10 % aufweist, bevor das erste Polymer abgebaut wird.

**7.** Vorrichtung nach einem der Ansprüche 1 bis 3, wobei das Lumen einen durchschnittlichen Gesamtdurchmesser zwischen 50 $\mu$m +/- 10 % und 120 $\mu$m +/- 10 %, vorzugsweise zwischen 60 $\mu$m +/- 10 % und 90 $\mu$m +/- 10 % aufweist, nachdem das erste Polymer abgebaut wurde.

**8.** Vorrichtung nach einem der Ansprüche 1 bis 7, wobei:

> (i) das erste, abbaubare Polymer, das zweite, langsamer abbaubare Polymer oder beide Polyglykolid, Poly(milchsäure), Poly(caprolacton), Chitosan, Poly(hydroxybuttersäure), Polyanhydride, Polyester, Polyphosphazene, Polyphosphoester, Polydioxanon, Poly(milch-co-glykolsäure) oder eine Mischung oder Copolymere davon umfassen; oder

(ii) das nicht abbaubare Polymer Poly(ethylenterephthalat), Poly(ethylen), Poly(propylen), Poly(tetrafluorethylen), Poly(methmethacrylat), Ethylen-co-vinylacetat, Poly(dimethylsiloxan), Poly(etherurethane), Poly(sulfon), Poly(ethylenoxid), Poly(ethylenoxid-co-propylenoxid), Poly(vinylalkohol) oder Copolymere davon umfasst.

9. Vorrichtung nach Anspruch 2, wobei die äußere Beschichtung Polyurethan, Silikon, Polydimethylsiloxan, Polytetrafluorethylen oder Copolymere davon umfasst.

10. Vorrichtung nach Anspruch 1, wobei die Vorrichtung eine schlauchförmige Struktur mit zumindest zwei Enden für den Durchfluss von Fluid durch das Lumen der Vorrichtung umfasst; wobei sich gegebenenfalls eine Fluiddruckdifferenz zwischen den beiden Enden der Vorrichtung von zwischen 0 mm Hg und 25 mm Hg +/- 10 % zu zwischen 0 mm Hg und weniger als 15 mm Hg ändert, wenn das Lumen an Größe zunimmt.

11. Vorrichtung nach einem der Ansprüche 1 bis 10, wobei die Vorrichtung eine Länge zwischen 0,5 mm +/-10 % und 30 mm +/- 10 %, vorzugsweise zwischen 1 mm +/-10 % und 20 mm +/- 10 % und noch bevorzugter zwischen 3 mm +/- 10 % und 10 mm +/- 10 % aufweist.

12. Vorrichtung nach einem der Ansprüche 1 bis 11, wobei die Wand eine Dicke zwischen 50 $\mu$m und 1 mm, vorzugsweise zwischen 100 $\mu$m +/- 10 % und 500 $\mu$m +/-10 % aufweist, wobei das erste Polymer gegebenenfalls eine Dicke der Wand zwischen 10 $\mu$m +/- 10 % und 80 $\mu$m +/- 10 % vor dem Abbau bildet.

13. Vorrichtung nach einem der Ansprüche 1 bis 12, wobei das erste Polymer und das zweite Polymer ein Massenverhältnis zwischen etwa 1:50 und etwa 10:1 aufweisen.

14. Vorrichtung nach Anspruch 3, wobei das eine oder die mehreren Mittel in einem Gewichtsprozentsatz von zwischen 0,09 % und 66 % in der Vorrichtung verkapselt, angegliedert oder gebunden sind.

15. Vorrichtung nach Anspruch 4, wobei die Vorrichtung durch sequentielles Extrudieren von zumindest zwei elektrisch geladenen Polymerlösungen, die gegebenenfalls jeweils ein therapeutisches, prophylaktisches oder diagnostisches Mittel umfassen, zu einer geerdeten Sammelvorrichtung hin hergestellt sind,

wobei die zwei Polymerlösungen Polymerfasern um die Sammelvorrichtung bilden und eine erste Polymerlösung ein abbaubares Polymer umfasst und eine zweite Polymerlösung ein im Vergleich zum ersten Polymer langsamer abbaubares Polymer oder ein nicht abbaubares Polymer umfasst,
wobei das Entnehmen der Polymerfasern aus der Sammelvorrichtung in der Wand der Vorrichtung resultiert, die ein Lumen umgibt,
und wobei das Lumen im Laufe der Zeit an Größe zunimmt, wenn die abbaubaren Polymerfasern abgebaut werden.

16. Vorrichtung nach einem der Ansprüche 1 bis 15 zur Verwendung in einem Verfahren zur Kontrolle der Verringerung des Augeninnendrucks eine Patienten, der dies benötigt, welches das Ableiten von Kammerwasser von innerhalb des Auges durch die Vorrichtung umfasst, wobei die Vorrichtung in einem Teil des Auges des Patienten implantiert ist; wobei der Patient gegebenenfalls ein Glaukom oder okuläre Hypertension hat.

17. Vorrichtung nach einem der Ansprüche 1 bis 15 zur Verwendung in einem Verfahren zum Heilen, Fixieren, Ersetzen oder Verbinden von Gewebe bei einem Patienten, der dies benötigt, welches das Implantieren der Vorrichtung in einen Teil des Körpers des Patienten umfasst; wobei die Vorrichtung gegebenenfalls implantiert ist, um Nervengewebe, Blutgefäße oder Bereiche des Auges zu ersetzen oder zu verbinden.

**Revendications**

1. Dispositif implantable comprenant une paroi entourant une lumière, dans lequel la paroi comprend

un premier polymère dégradable, formant la surface luminale de la paroi,
et un second polymère non dégradable ou dégradable plus lentement, par rapport au premier polymère dégradable, formant la surface extérieure de la paroi,

dans lequel la paroi est formée de nanofibres polymères,

dans lequel les nanofibres sont alignées et durcies par chauffage à une température supérieure à la température de transition vitreuse du polymère pour augmenter la résistance mécanique et la flexibilité par rapport au polymère qui n'est pas aligné et durci, et

dans lequel la lumière s'agrandit au fil du temps à mesure que le premier polymère dégradable se dégrade.

2. Dispositif selon la revendication 1, dans lequel le dispositif comprend un revêtement sur la surface extérieure de la paroi.

3. Dispositif selon la revendication 1 ou la revendication 2, dans lequel le dispositif comprend en outre un ou plusieurs agents thérapeutiques, prophylactiques ou diagnostiques.

4. Dispositif selon l'une quelconque des revendications 1 à 3, dans lequel les nanofibres sont fabriquées par électrofilage.

5. Dispositif selon l'une quelconque des revendications 1 à 3, dans lequel le premier polymère se dégrade sur une période de temps comprise entre environ 2 jours et environ 100 jours *in vivo.*

6. Dispositif selon l'une quelconque des revendications 1 à 3, dans lequel la lumière a un diamètre global moyen compris entre 40 $\mu$m +/- 10 % et 100 $\mu$m +/- 10 %, de préférence entre 50 $\mu$m +/- 10 % et 80 $\mu$m +/- 10 %, et plus préférablement de 50 $\mu$m +/-10 %, avant la dégradation du premier polymère.

7. Dispositif selon l'une quelconque des revendications 1 à 3, dans lequel la lumière a un diamètre global moyen compris entre 50 $\mu$m +/- 10 % et 120 $\mu$m +/- 10 %, de préférence entre 60 $\mu$m +/- 10 % et 90 $\mu$m +/- 10 %, après la dégradation du premier polymère.

8. Dispositif selon l'une quelconque des revendications 1 à 7, dans lequel :

(1) le premier polymère dégradable, le second polymère dégradable plus lentement, ou les deux, comprennent du polyglycolide, du poly(acide lactique), de la poly(caprolactone), du chitosane, du poly(acide hydroxybutyrique), des polyanhydrides, des polyesters, des polyphosphazènes, des polyphosphoesters, de la polydioxanone, du poly(acide lactique-co-glycolique), ou un mélange ou des copolymères de ceux-ci ; ou

(ii) le polymère non dégradable comprend du poly(téréphtalate d'éthylène), du poly(éthylène), du poly(propylène), du poly(tétrafluoroéthylène), du poly(méthacrylate de méthyle), de l'éthylène-co-acétate de vinyle, du poly(diméthylsiloxane), des poly(éther-uréthanes), de la poly(sulfone), du poly(oxyde d'éthylène), du poly(oxyde d'éthylène-co-oxyde de propylène), du poly(alcool vinylique), ou des copolymères de ceux-ci.

9. Dispositif selon la revendication 2, dans lequel le revêtement extérieur comprend du polyuréthane, du silicone, du polydiméthylsiloxane, du polytétrafluoroéthylène ou des copolymères de ceux-ci.

10. Dispositif selon la revendication 1, dans lequel le dispositif a une structure tubulaire avec au moins deux extrémités pour le passage de fluide à travers la lumière du dispositif ; éventuellement dans lequel une différence de pression de fluide entre les deux extrémités du dispositif change d'entre 0 mm Hg et 25 mm Hg +/- 10 % à entre 0 mm Hg et moins de 15 mm Hg à mesure que la lumière s'élargit.

11. Dispositif selon l'une quelconque des revendications 1 à 10, dans lequel le dispositif a une longueur comprise entre 0,5 mm +/- 10 % et 30 mm +/-10 %, de préférence entre 1 mm +/- 10 % et 20 mm +/-10 %, et plus préférablement entre 3 mm +/- 10 % et 10 mm +/- 10 %.

12. Dispositif selon l'une quelconque des revendications 1 à 11, dans lequel la paroi a une épaisseur comprise entre 50 $\mu$m et 1 mm, de préférence entre 100 $\mu$m +/- 10 % et 500 $\mu$m +/- 10 %, éventuellement dans lequel le premier polymère forme une épaisseur de la paroi comprise entre 10 $\mu$m +/- 10 % et 80 $\mu$m +/- 10 % avant dégradation.

13. Dispositif selon l'une quelconque des revendications 1 à 12, dans lequel le premier polymère et le second polymère ont un rapport de masse compris entre environ 1:50 et environ 10:1.

14. Dispositif selon la revendication 3, dans lequel les un ou plusieurs agents sont encapsulés, associés ou liés dans le dispositif à un pourcentage pondéral compris entre 0,09 % et 66 %.

**15.** Dispositif selon la revendication 4, dans lequel le dispositif est préparé en extrudant séquentiellement au moins deux solutions polymères chargées électriquement, chacune comprenant éventuellement un agent thérapeutique, prophylactique ou diagnostique, vers un collecteur mis à la terre,

dans lequel les deux solutions polymères forment des fibres polymères autour du collecteur, et une première solution polymère comprend un polymère dégradable et une seconde solution polymère comprend un polymère dégradable plus lentement, par rapport au premier polymère, ou un polymère non dégradable, dans lequel l'élimination des fibres polymères du collecteur permet à la paroi du dispositif d'entourer une lumière, et dans lequel la lumière s'agrandit au fil du temps à mesure que les fibres polymères dégradables se dégradent.

**16.** Dispositif selon l'une quelconque des revendications 1 à 15 destiné à être utilisé dans un procédé de contrôle de la réduction de la pression intraoculaire chez un patient en ayant besoin, comprenant le détournement de l'humeur aqueuse de l'intérieur d'un œil à travers ledit dispositif, dans lequel le dispositif est implanté dans une partie de l'œil du patient ; éventuellement dans lequel le patient souffre de glaucome ou d'hypertension oculaire.

**17.** Dispositif selon l'une quelconque des revendications 1 à 15 destiné à être utilisé dans un procédé de cicatrisation, de fixation, de remplacement ou de liaison de tissu chez un patient en ayant besoin, comprenant l'implantation dudit dispositif dans une partie du corps du patient ; éventuellement dans lequel le dispositif est implanté pour remplacer ou relier un tissu nerveux, le système vasculaire ou des compartiments de l'œil.

FIG. 1

FIG. 2

FIG. 3

EP 3 426 315 B1

FIG. 4

FIG. 5A

FIG. 5B

FIGS. 6A-6C

FIG. 7A

FIG. 7B

FIG. 8A

FIG. 8B

# EP 3 426 315 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013184538 A
- WO 9933410 A
- US 2015025031 W, Ibrahim Z
- US 6214901 B
- US 7135009 B

### Non-patent literature cited in the description

- **QUIGLEY HA ; BROMAN AT**. *Br J Ophthalmol*, 2006, vol. 90 (3), 262-267
- **THAM Y-C et al.** *Ophthalmology*, 2014, vol. 121 (11), 2081-90
- **SACCÀ SC et al.** Handbook of Nutrition, Diet and the Eye. Academic, 2014, 29-40
- **LESKE MC et al.** *Ophthalmology*, 1999, vol. 106 (11), 2144-53
- **GEDDE SJ et al.** *Am J Ophthalmol.*, 2009, vol. 148 (5), 670-684
- **GEDDE SJ et al.** *Am J Ophthalmol.*, 2007, vol. 143 (1), 23-31
- **SOLUS JF et al.** *Ophthalmology*, 2012, vol. 119 (4), 703-711
- **OLAYANJU JA et al.** *JAMA Ophthalmol.*, 2015, vol. 133 (5), 574-580
- **FEINER L ; PILTZ-SEYMOUR JR**. *Curr Opin Ophthalmol.*, 2003, vol. 14 (2), 106-111
- **JAMPEL HD et al.** *Am J Ophthalmol.*, 2005, vol. 140 (1), 16-22
- **BUDENZ DL et al.** *Ophthalmology*, 2015, vol. 122 (2), 308-316
- **CHENG J et al.** *J Glaucoma*, 2015, vol. 24 (4), e34-5
- **FREEDMAN J**. *Ophthalmology*, 2009, vol. 116 (1), 166
- **PISTNER et al.** *Biomaterials*, 1993, vol. 14, 291-298
- **LI D et al.** *Advanced materials*, 2004, vol. 16 (14), 1151-1170
- **BHARDWAJ N et al.** *Biotechnol Adv.*, 2010, vol. 28 (3), 325-347
- **LI D ; XIA Y.** *Advanced Materials*, 2004, vol. 16 (14), 1151-1170
- **BHARDWAJ N ; KUNDU SC.** *Biotechnol Adv.*, 2010, vol. 28 (3), 325-347
- **NAYAK R et al.** *Textile Research Journal*, 2011, vol. 82 (2), 129-147
- **PRASAD SHASTRI**. *Current Pharmaceutical Bio-technology*, 2003, vol. 4, 331-337
- Remington's Pharmaceutical Sciences. Lippincott Williams & Wilkins, 2000, 704
- **KHAN W. et al.** Focal Controlled Drug Delivery. Springer US, 2014, 33-59
- **LAVOISIER A et al.** *MAbs*, 2015, vol. 7 (1), 77-83
- **FU J et al.** *Molecular Pharmaceutics*, 2016, vol. 13 (9), 2987-2995
- **SHEYBANI A et al.** *Invest Ophthalmol Vis Sci.*, 2015, vol. 56 (8), 4789-4795
- **WONG S-C et al.** *Polymer*, 2008, vol. 49 (21), 4713-4722
- **KAM KR et al.** *Tissue Eng Part A*, 2014, vol. 20 (1-2), 130-138
- **RICHTER GM et al.** *Clin Ophthalmol.*, 2016, vol. 10, 189-206
- **HARAKE RS et al.** *Ann Biomed Eng.*, vol. 43 (10), 2394-2405
- **KHAN W et al.** Focal Controlled Drug Delivery. Springer US, 2014, 33-59
- **SHEYBANI A et al.** *J Cataract Refract Surg.*, 2015, vol. 41 (9), 1905-1909
- **SHEYBANI A et al.** *J Glaucoma*, 2016, vol. 25 (7), e691-6
- **SHARP KV et al.** *Experiments in Fluid*, 2004, vol. 36 (5), 741-747
- **ECHAVEZ M et al.** *Philipp J Ophthalmol.*, 2012, vol. 37 (1), 45-51
- **ACOSTA AC et al.** *Archives of Ophthalmology*, 2006, vol. 124 (12), 1742-1749
- **BATLLE JF et al.** *J. of Glaucoma*, 2016, vol. 25 (2), e58-e65